(19) Europäisches Patentamt
European Patent Office
Office européen des brevets

(11) EP 3 501 521 A1

(12) EUROPEAN PATENT APPLICATION

(43) Date of publication:
**26.06.2019 Bulletin 2019/26**

(21) Application number: **18174583.7**

(22) Date of filing: **16.02.2012**

(51) Int Cl.:
***A61K 31/573*** $^{(2006.01)}$ ***A61K 31/65*** $^{(2006.01)}$
***A61K 31/282*** $^{(2006.01)}$ ***A61K 31/4439*** $^{(2006.01)}$
***A61K 31/506*** $^{(2006.01)}$ ***A61K 31/416*** $^{(2006.01)}$
***A61K 31/24*** $^{(2006.01)}$ ***A61K 9/10*** $^{(2006.01)}$
***A61K 47/10*** $^{(2017.01)}$ ***A61P 27/16*** $^{(2006.01)}$

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2011 US 201161444413 P**
**02.08.2011 US 201161514272 P**

(62) Document number(s) of the earlier application(s) in accordance with Art. 76 EPC:
**12746960.9 / 2 675 409**

(27) Previously filed application:
**16.02.2012 PCT/US2012/025511**

(71) Applicant: **Otonomy, Inc.**
**San Diego, CA 92121 (US)**

(72) Inventors:
- **Fabrice, PIU**
**San Diego, California 92131 (US)**
- **Qiang, YE**
**San Diego, California 92130 (US)**
- **Luis, DELLAMARY**
**San Marcos, California 92069 (US)**
- **Carl, LEBEL**
**Malibu, California 90265 (US)**

(74) Representative: **Campabadal i Monfà, Gemma**
**Wilson Sonsini Goodrich & Rosati LLP**
**Rue Montoyer 47**
**1000 Bruxelles (BE)**

Remarks:
•This application was filed on 28-05-2018 as a divisional application to the application mentioned under INID code 62.
•Claims filed after the date of receipt of the application (Rule 68(4) EPC).
•Claims 16-34 are deemed to be abandoned due to non-payment of the claims fees (Rule 45(3) EPC).

# (54) PREVENTION OF AND RECOVERY FROM DRUG-INDUCED OTOTOXICITY

(57) Provided herein are methods for preventing and/or reducing the severity of drug induced ototoxicity. Provided herein are methods for recovery from hearing loss due to drug-induced ototoxicity.

EP 3 501 521 A1

## Description

### CROSS REFERENCE

This application claims the benefit of U.S. Provisional Application No. 61/444,413, filed February 18, 2011; and U.S. Provisional Application No. 61/514,272, filed August 2, 2011; and each application is incorporated herein by reference in its entirety.

### BACKGROUND OF THE INVENTION

Several therapeutic agents cause ototoxicity. Damage to the inner ear results in loss of cochlear hair cells, cells of the stria vascularis and/or the spiral ganglion, ultimately leading to hearing loss.

### SUMMARY OF THE INVENTION

Provided herein are compositions and methods for preventing drug-induced ototoxicity and/or reversing hearing loss due to drug-induced ototoxicity. Ototoxicity is often a side effect of certain treatment regimens (e.g., chemotherapy, use of aminoglycoside antibiotics, salicylates or the like). In some embodiments, the methods provided herein allow for continued use of agents that would otherwise cause the side-effect of hearing loss and/or would be discontinued due to ototoxicity. Where ototoxicity is dose-limiting for a drug (e.g., a chemotherapeutic agent, an aminoglycoside antibiotic or the like), the methods provided herein prevent onset of drug induced ototoxic side-effects, thereby allowing for use of higher doses of the drug and/or a better treatment outcome for a patient undergoing therapy with the ototoxic drug. In some other embodiments, the methods provided herein allow for recovery of hearing in a patient who has already undergone treatment with a ototoxicity-inducing drug with the intent of recovering and/or reversing the hearing loss associated with previous regimen(s) of the ototoxic drug

In one aspect, provided herein is a method for preventing drug-induced ototoxicity in an individual in need thereof comprising intratympanic administration of a pharmaceutical composition comprising a multiparticulate corticosteroid to the individual in need thereof, wherein the pharmaceutical composition is administered prior to onset of therapy with the drug, and wherein the composition provides sustained release of the corticosteroid into the ear for a period of at least 5 days after a single administration.

In a different aspect, provided herein are methods for recovery of hearing or reversal of hearing loss from drug-induced ototoxicity in an individual in need thereof comprising intratympanic administration of a pharmaceutical composition comprising a multiparticulate corticosteroid to the individual in need thereof, wherein the pharmaceutical composition is administered to an individual after a treatment course with the ototoxicity-inducing drug, and wherein the composition provides sustained release of the corticosteroid into the ear for a period of at least 5 days after a single administration.

In some embodiments of the method described above, the drug-induced ototoxicity is hearing loss. In another embodiment, the drug-induced ototoxicity is chemotherapy-induced ototoxicity.

In some embodiments of the method described above, the chemotherapeutic agent that induces ototoxicity is a platinum based chemotherapeutic agent, a bis-platinate, vincristine, an aminoglycoside antibiotic, a macrolide antibiotic, a diuretic or a salicylate.

In some embodiments of the method described above, the platinum based chemotherapeutic agent is cis-platin, carboplatin or oxiplatin.

In some embodiments of the method described above, the bis-platinate is CT-47613 or CT-47609.

In some embodiments of the method described above, the chemotherapeutic agent that induces ototoxicity is vincristine.

In some embodiments of the method described above, the aminoglycoside antibiotic is gentamicin, streptomycin, kanamycin, amikacin or neomycin.

In some embodiments of the method described above, the macrolide antibiotic is erythromycin, azithromycin or clindamycin.

In some embodiments of the method described above, the intratympanic composition comprises a gel or a viscous preparation. In some embodiments of the method described above, the intratympanic composition comprises a gel. In some embodiments of the method described above, the intratympanic composition comprises a viscous preparation.

In some embodiments of the method described above, the intratympanic composition comprises a thermoreversible gel.

In some embodiments of the method described above, the thermoreversible gel comprises a copolymer of polyoxyethylene and polyoxypropylene in an amount sufficient to provide a gelation temperature of between about 15

°C and about 42 °C.

In some embodiments of the method described above, the corticosteroid is selected from dexamethasone, dexamethasone acetate, prednisone and methylprednisolone, or pharmaceutically acceptable salt thereof. In some embodiments, the multiparticulate corticosteroid is essentially in the form of micronized particles.

Provided herein in one aspect is a method for preventing hearing loss due to acoustic trauma in an individual in need thereof comprising intratympanic administration of a pharmaceutical composition comprising a multiparticulate JNK inhibitor to the individual in need thereof, wherein the pharmaceutical composition is administered prior to onset of acoustic trauma, and wherein the composition provides sustained release of the JNK inhibitor into the ear for a period of at least 5 days after a single administration.

In some embodiments of the method described above, the JNK inhibitor is selected from minocycline; SB-203580 (4-(4-Fluorophenyl)-2-(4-methylsulfinyl phenyl)-5-(4-pyridyl) 1H-imidazole); PD 169316 (4-(4-Fluorophenyl)-2-(4-nitrophenyl)-5-(4-pyridyl)-1H-imidazole); SB 202190 (4-(4-Fluorophenyl)-2-(4-hydroxyphenyl)-5-(4-pyridyl)1H-imidazole); RWJ 67657 (4-[4-(4-fluorophenyl)-1-(3-phenylpropyl)-5-(4-pyridinyl)-1H-imidazol -2-yl]-3-butyn-1-ol); SB 220025 (5-(2-Amino-4-pyrimidinyl)-4-(4-fluorophenyl)-1-(4-piperidinlyl)imidazole); AM-111; and SP600125.

In some embodiments of the method described above, the JNK inhibitor is SP600125.

In some embodiments of the method described above, the intratympanic composition comprises a gel or a viscous preparation. In some embodiments of the method described above, the intratympanic composition comprises a gel. In some embodiments of the method described above, the intratympanic composition comprises a viscous preparation.

In some embodiments of the method described above, the intratympanic composition comprises a thermoreversible gel.

In some embodiments of the method described above, the thermoreversible gel comprises a copolymer of polyoxyethylene and polyoxypropylene polyoxypropylene in an amount sufficient to provide a gelation temperature of between about 15 °C and about 42 °C. In some embodiments, the multiparticulate JNK inhibitor is essentially in the form of micronized particles.

In some embodiments of the method described above, the intratympanic composition comprises a non-thermoreversible gel. In some embodiments, the gel comprises a viscosity-enhancing polymer or a gel-forming polymer. In some embodiments, the viscosity-enhancing polymer is selected from hyaluronic acid or salts thereof. In some embodiments, the gel-forming polymer are co-polymers comprising lactic acid and glycolic acid monomers, including PLGA or poly(lactic-co-glycolic acid).

Provided herein, in some embodiments, are methods for preventing drug-induced ototoxicity in an individual in need thereof comprising intratympanic administration of a pharmaceutical composition comprising a thermoreversible gel and a multiparticulate corticosteroid to the individual in need thereof, wherein the pharmaceutical composition is administered prior to onset of therapy with the drug, and wherein the composition provides sustained release of the corticosteroid into the ear for a period of at least 5 days after a single administration.

In a different aspect, provided herein are methods for recovery of hearing or reversal of hearing loss from drug-induced ototoxicity in an individual in need thereof comprising intratympanic administration of a pharmaceutical composition comprising a thermoreversible gel and a corticosteroid to the individual in need thereof, wherein the pharmaceutical composition is administered to an individual after a treatment course with the ototoxicity-inducing drug, and wherein the composition provides sustained release of the corticosteroid into the ear for a period of at least 5 days after a single administration.

In some embodiments, the ototoxicity is hearing loss. In some embodiments, the drug-induced ototoxicity is chemotherapy-induced ototoxicity. In some embodiments, the chemotherapeutic agent that induces ototoxicity is a platinum based chemotherapeutic agent. In some embodiments, the platinum based chemotherapeutic agent is cisplatin, carboplatin or oxiplatin. In some embodiments, the platinum based chemotherapeutic agent is a bis-platinate. In some embodiments, the bis-platinate is CT-47613 or CT-47609. In some embodiments, the chemotherapeutic agent that induces ototoxicity is vincristine.

In some embodiments, the drug that induces ototoxicity is an aminoglycoside antibiotic. In some embodiments, the aminoglycoside antibiotic is gentamicin, streptomycin, kanamycin, amikacin or neomycin.

In some embodiments, the drug that induces ototoxicity is a macrolide antibiotic. In some embodiments, the macrolide antibiotic is erythromycin, azithromycin or clindamycin. In some embodiments, the drug-induced ototoxicity is induced by diuretics or salicylates.

In some embodiments, the thermoreversible gel comprises a copolymer of polyoxyethylene and polyoxypropylene. In some embodiments, the copolymer of polyoxyethylene and polyoxypropylene is Poloxamer 407. In some embodiments, the corticosteroid comprises multiparticulates. In some embodiments, the corticosteroid is essentially in the form of micronized particles. In some embodiments, the corticosteroid is selected from dexamethasone, dexamethasone acetate, prednisone and methylprednisolone, or pharmaceutically acceptable salt thereof.

In one aspect, provided herein are methods for preventing hearing loss due to acoustic trauma in an individual

in need thereof comprising intratympanic administration of a pharmaceutical composition comprising a thermoreversible gel and a JNK inhibitor to the individual in need thereof, wherein the pharmaceutical composition is administered prior to onset of acoustic trauma, and wherein the composition provides sustained release of the JNK inhibitor into the ear for a period of at least 2 days after a single administration.

In some embodiments, the composition provides sustained release of the JNK inhibitor into the ear for a period of at least 3 days. In some embodiments, the composition provides sustained release of the JNK inhibitor into the ear for a period of at least 4 days. In some embodiments, the composition provides sustained release of the JNK inhibitor into the ear for a period of at least 5 days.

In some embodiments, the JNK inhibitor is selected from minocycline; SB-203580 (4-(4-Fluorophenyl)-2-(4-methylsulfinyl phenyl)-5-(4-pyridyl)1H-imidazole); PD 169316 (4-(4-Fluorophenyl)-2-(4-nitrophenyl)-5-(4-pyridyl)-1H-imidazole); SB 202190 (4-(4-Fluorophenyl)-2-(4-hydroxyphenyl)-5-(4-pyridyl)1H-imidazole); RWJ 67657 (4-[4-(4-fluorophenyl)-1-(3-phenylpropyl)-5-(4-pyridinyl)-1H-imidazol -2-yl]-3-butyn-1-ol); SB 220025 (5-(2-Amino-4-pyrimidinyl)-4-(4-fluorophenyl)-1-(4-piperidinlyl)imidazole); AM-111; and SP600125. In some embodiments, the JNK inhibitor is SP600125.

In some embodiments, the thermoreversible gel comprises a copolymer of polyoxyethylene and polyoxypropylene. In some embodiments, the copolymer of polyoxyethylene and polyoxypropylene is Poloxamer 407. In some embodiments, the JNK inhibitor comprises multiparticulates. In some embodiments, the JNK inhibitor is essentially in the form of micronized particles.

In another aspect, provided herein is an intratympanic composition (e.g., any otic composition described above and below) for use in prophylactic treatment of ototoxicity. In some embodiments, the ototoxicity is hearing loss as described above or below. In some embodiments, the ototoxicity is chemotherapy-induced ototoxicity as described above or below.

In another aspect, provided herein is an intratympanic composition (e.g., any otic composition described above and below) for use in prophylactic treatment of acoustic trauma induced hearing loss. In another aspect, provided herein is an intratympanic composition (e.g., any otic composition described above and below) for use in recovery of hearing or reversal of hearing loss from drug-induced ototoxicity.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**Figure 1** illustrates that a composition comprising a theremoreversible gel and dexamethasone is protective against Cisplatin induced hearing loss wherein the composition is administered to guinea pigs 24 hours prior to cisplatin treatment.

**Figure 2** illustrates the protective effect of compositions comprising a theremoreversible gel and dexamethasone in guinea pigs with noise induced hearing loss wherein the composition is administered to guinea pigs prior to exposure to noise.

**Figure 3** illustrates the protective effect of a composition comprising a theremoreversible gel and dexamethasone in guinea pigs exposed to increasing levels of acoustic trauma wherein the composition is administered to guinea pigs prior to exposure to acoustic trauma.

**Figure 4** illustrates that a composition comprising a theremoreversible gel and dexamethasone is superior to DSP solution for protection against acute acoustic trauma when the composition is administered prior to exposure to acoustic trauma.

**Figure 5** illustrates that a composition comprising a theremoreversible gel and a JNK inhibitor (anti-apoptotic agent) protects prophylactically against acute acoustic trauma.

**Figure 6** illustrates that a composition comprising a theremoreversible gel and IGF-1 protects against acoustic trauma.

**Figure 7** illustrates inner ear exposure of IGF-1 up on intratympanic administration of a formulation described herein.

**Figure 8** illustrates effect of a sustained release hydrogel formulation of dexamethasone in an acute cisplatin

ototoxicity paradigm. Guinea pigs (n=6) received a single bilateral intratympanic injection varying concentrations of dexamethasone one day prior to a single injection of cisplatin (12 mg/kg). Auditory function was assessed at Day 7 post cisplatin treatment.

**Figure 9** illustrates treatment of guinea pigs in an acute cisplatin ototoxicity model with a 2.0% DSP solution. Guinea pigs (n=6) received a single bilateral intratympanic injection of either saline (white bars) or 2.0% DSP solution (black bars). One day later, animals were given a single administration of cisplatin (12 mg/kg). Auditory function was assessed at Day 7 post cisplatin treatment. No significant differences between control and treated animals were observed.

**Figure 10** illustrates effect of Mifepristone, a GR and MR antagonist. Mifeprestone antagonizes dexamethasone gel protection against acute cisplatin ototoxicity. Guinea pigs (n=6) received a single bilateral intratympanic injection of either poloxamer vehicle (white bars), 6.0% mifepristone (light grey bars), 6.0% dexamethasone gel (dark grey bars) or 6.0% dexamethasone + 6.0% mifepristone (black bars). One day later, animals were treated with cisplatin (12 mg/kg). Auditory function was assessed at the indicated times.

**Figure 11** illustrates effect of dexamethasone gel against chronic exposure to cisplatin. Guinea pigs (n=6) received three bilateral intratympanic injections at a one-week interval of either poloxamer vehicle (circles) or 6.0% dexamethasone (squares). Each of these injections were followed 30 min later by cisplatin administration (4 mg/kg). Auditory function was assessed at the indicated times.

## DETAILED DESCRIPTION OF THE INVENTION

A wide variety of drugs are ototoxic. Factors affecting ototoxicity include dose, duration of therapy, concurrent renal failure, infusion rate, lifetime dose, co-administration with other drugs having ototoxic potential, and/or genetic susceptibility. In cases where hearing loss is inevitable due to cumulative ototoxic exposures, patients need to be cognizant of the tradeoffs of potentially curative therapy versus permanent hearing loss. There is a need for treatment regimens that minimize this complication. Accordingly, provided herein are prophylactic methods and/or treatment regimens that prevent or delay onset of drug-induced ototoxicity and exert an otoprotective effect. Advantageously, the methods described herein comprise localized administration to the ear thereby avoiding interference with the therapeutic efficacy of the systemically administered otoxicity-inducing drugs (e.g., chemotherapeutic drugs, aminoglycoside antibiotics and the like).

### Otoxicity and inner ear damage

The inner ear comprises two parts: the osseous labyrinth and the membranous labyrinth. The vestibule, the semicircular canals and the cochlea form the osseous labyrinth. The osseous labyrinth is filled with the perilymph which also surrounds the soft tissue of the membranous labyrinth. The membranous labyrinth contains a series of closed sacs containing the endolymph.

The vestibule connects the cochlea in front with the semicircular canals at the back. The cochlea is a conical and spiraled structure located in the rostral part of the labyrinth. The cochlear duct is a single bony tube approximately 34 mm long in humans and spirals around a middle core that contains the spiral ganglion of the auditory nerve. The cochlear duct is divided into three chambers called scalae: the scala vestibule, the scala media and the scala tympani. The oval window touches the scala vestibule and the round window touches the scala tympani. The organ of Corti is the sensory epithelium of the cochlea and comprises rod-shaped cells, supporting cells, and hair cells.

Human ears contain about 17,000 hair cells: a single row of inner hair cells long the length of the cochlea and three rows of outer hair cells extending from the base to the apex of the cochlea. The distribution of receptor cells in the ear is sparse when compared to other sensory organs such as the retina or nasal epithelium; hence the loss of even a few thousand hair cells results in severe hearing loss. Any cochleo-vestibular ototoxicity or acoustic trauma affects hair cells profoundly; humans cannot regenerate hair cells and once a cochlear hair cell is damaged, the reduction in hearing is permanent. Accordingly, provided herein are methods that protect hair cells and/or prevent ototoxic damage to hair cells. Also provided herein are methods that prevent damage to hair cells from acoustic trauma. Further provided herein are methods that allow for recovery of hearing following hearing loss and/or inner ear damage.

### Ototoxicity inducing drugs

Platinum based chemotherapeutic agents

Platinum based compounds are commonly used as antineoplastic agents. Examples of platinum based chemotherapeutic agents include cis-platin, carboplatin or oxiplatin. Other platinum based chemotherapeutic agents include the bis-platinates. Examples of bis-platinates include and are not limited to CT-47613 and CT-47609.

Platinum-based drugs induce ototoxicity which manifests as sensorineural hearing loss with or without tinnitus. For example, children with neuroblastoma receive high-dose carboplatin as part of their conditioning regimen for autologous marrow transplantation and have a high incidence of speech frequency hearing loss. Ototoxicity is dose-related and cumulative. When ototoxicity develops, treatment of cancer with platinum based drugs is stopped or a less potent antineoplastic agent is used. This affects treatment outcome for cancer patients. There is currently no treatment available for platinum-based chemotherapeutic- induced ototoxicity. Attempts to co-administer systemic antioxidants with cisplatin treatment have not been successful because the antioxidants inhibit the antineoplastic effect of cisplatin or exhibit toxicities of their own. *See* for example, Rybak et Al., Drug Disc. Today 2005, 10:1313-21.

Accordingly provided herein are methods for pretreatment of cancer patients in need of carboplatin and/or cisplatin and/or oxiplatin therapy comprising administration of an intratympanic injection of a composition comprising a theremoreversible gel and a corticosteroid such that the composition exerts an otoprotective effect and prevents ototoxicity induced by platinum-containing chemotherapeutic agents. In some of such embodiments, the composition provides sustained release of the corticosteroid into the cochlea for at least 5 days after a single adminstration.

Other anticancer agents

Other anticancer drugs that cause ototoxicity at high doses include, for example, vincristine. Accordingly also contemplated within the scope of embodiments presented herein are methods for preventing ototoxicity in individuals in need of chemotherapy (e.g., vincristine treatment) comprising administration of an intratympanic injection of a composition comprising a theremoreversible gel and a corticosteroid such that the composition exerts an otoprotective effect and prevents ototoxicity.

Aminoglycoside antibiotics

Certain aminoglycoside antibiotics are associated with ototoxic side effects. Streptomycin causes damage to the vestibular portion of the inner ear. Although vertigo and difficulty maintaining balance tend to be temporary, severe loss of vestibular sensitivity persists, sometimes permanently. Loss of vestibular sensitivity causes difficulty walking, especially in the dark, and oscillopsia (a sensation of bouncing of the environment with each step). About 4 to 15% of patients who receive 1 g/day for > 1 wk develop measurable hearing loss, which usually occurs after a short latent period (7 to 10 days) and slowly worsens if treatment is continued. Complete, permanent deafness may follow.

Neomycin, kanamicin and amikacin are cochleotoxic and cause profound, permanent hearing loss while sparing balance. Viomycin has both cochlear and vestibular toxicity. Gentamicin and tobramycin cause vestibular and cochlear toxicity, causing impairment in balance and hearing. The aminoglycoside Vancomycin causes hearing loss, often in the presence of renal insufficiency.

Aminoglycoside ototoxicity causes irreversible damage to the outer hair cells at the basal turn of the cochlea. There is currently no treatment available for aminoglycoside ototoxicity. Accordingly provided herein are methods for preventing ototoxicity in individuals in need of treatment with aminoglycoside antibiotics comprising administration of an intratympanic injection of a composition comprising a theremoreversible gel and a corticosteroid such that the composition exerts an otoprotective effect and prevents ototoxicity induced by an aminoglycoside antibiotic. In some of such embodiments, the composition provides sustained release of the corticosteroid into the cochlea for at least 5 days after a single administration.

Other antibiotics

Erythromycin, azithromycin and clindamycin are macrolide antibiotics that cause hearing loss in some individuals. Accordingly, also contemplated within the scope of embodiments presented herein are methods for preventing ototoxicity in individuals in need of treatment with antibiotics that induce ototoxicity comprising administration of an intratympanic injection of a composition comprising a theremoreversible gel and a corticosteroid such that the composition exerts an otoprotective effect and prevents ototoxicity induced by the drug.

Diuretics and Salicylates

Certain diuretics such as ethacrynic acid, or furosemide cause profound and permanent hearing loss. Salicylates in high doses, and the antimalarial drug quinine are also associated with temporary hearing loss. Accordingly, also contemplated within the scope of embodiments presented herein are methods for preventing ototoxicity in individuals in need of treatment with diuretics (including loop diuretics), salicylates and/or any other ototoxic agent comprising administration of an intratympanic injection of a composition comprising a theremoreversible gel and a corticosteroid such that the composition exerts an otoprotective effect and prevents ototoxicity induced by the drug.

**Acoustic trauma**

Provided herein are methods for preventing hearing loss due to acoustic trauma in an individual in need thereof comprising intratympanic administration of a pharmaceutical composition comprising a thermoreversible gel and a corticosteroid to the individual in need thereof, wherein the pharmaceutical composition is administered prior to onset of acoustic trauma, and wherein the composition provides sustained release of the corticosteroid into the ear (e.g., the cochlea) for a period of at least 5 days after a single administration.

In some instances, acoustic trauma causes hair cell damage resulting in permanent hearing loss. A variety of environmental sources cause acoustic trauma including, and not limited to, the noise of jet planes (e.g., near an airport), noise of artillery and/or gunfire and/or bombs (e.g., in a war zone), noise of heavy machinery (e.g., in a factory, on an oil platform), loud music (e.g., at a rock concert) and the like.

In specific embodiments, administration of a composition described herein (e.g., a thermoreversible gel composition comprising a copolymer of polyoxyethylene or polyoxypropylene and a corticosteroid or a JNK inhibitor) prior to exposure to loud noise has a protective effect and prevents hair cell damage and/or hearing loss. Thus, in an exemplary embodiment, administration of a composition described herein to an individual prior to deployment in a war zone prevents and/or reduces the severity of hearing loss.

**Active agents**

Corticosteroids

In some embodiments of the methods described herein, the otic formulations comprise corticosteroids (including agents that act at glucocorticoid receptors) or other anti-inflammatory steroids that are compatible with the formulations disclosed herein. One advantage of the use of the methods described herein is the greatly reduced systemic exposure to anti-inflammatory glucocorticoid steroids.

In one embodiment is the active pharmaceutical ingredient of a formulation described herein is prednisolone. In another embodiment the active pharmaceutical ingredient of the formulation described herein is dexamethasone. In an additional embodiment, the active pharmaceutical ingredient of the formulation described herein is beclomethasone. In an additional embodiment, the active pharmaceutical ingredient of the formulation described herein is triamcinolone. In a further embodiment, the active pharmaceutical ingredient of the formulation described herein is selected from 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, or combinations thereof.

Non-steroidal anti-inflammatory agents include and are not limited to Ibuprofen, Naproxen, Fenoprofen, Ketoprofen, Flurbiprofen, Oxaprozin, Loxoprofen, Indomethacin, Sulindac, Etodolac, Ketorolac, Diclofenac, Nabumetone, Piroxicam, Meloxicam, Tenoxicam, Droxicam, Lornoxicam, Isoxicam, Fenamic acid derivatives, Mefenamic acid, Meclofenamic acid, Flufenamic acid, Tolfenamic acid, Celecoxib, Rofecoxib, Valdecoxib, Parecoxib, Lumiracoxib, Etoricoxib, Firocoxib, Sulphonanilides, Nimesulide, and the like.

Other agents suitable for compositions described herein include and are not limited to Prednisone, Fluticasone propionate (S-(fluoromethyl) (6S,8S,9R,10S,11S,13S,14S,16R,17R)-6,9-difluoro-11,17-dihydroxy-10,13,16-trimethyl-3-oxo-6,7,8,11,12,14,15,16-octahydrocyclopenta[a]phenanthrene-17-carbothioate), mometasone furoate ((11β,16α)-9,21-dichloro-11-hydroxy-16-methyl-3,20-dioxopregna-1,4-dien-17-yl 2-furoate), and the like.

Corticosteroids and/or non-steroidal anti-inflammatory agents that are not disclosed herein but which are useful in sustained release formulations and methods described herein are expressly included and intended within the scope of the methods presented.

In some embodiments, the compositions described herein provide sustained release of a corticosteroid into the cochlea after a single administration of the composition. In some of such embodiments, the compositions described herein provide sustained release of a corticosteroid into the cochlea for at least 5 days. In some of such embodiments, the compositions described herein provide sustained release of a corticosteroid into the cochlea for at least 6 days. In some of such embodiments, the compositions described herein provide sustained release of a corticosteroid into the cochlea for at least 7 days. In some of such embodiments, the compositions described herein provide sustained release

of a corticosteroid into the cochlea for at least 8 days. In some of such embodiments, the compositions described herein provide sustained release of a corticosteroid into the cochlea for at least 9 days. In some of such embodiments, a compositions described herein provide sustained release of a corticosteroid into the cochlea for at least 10 days. In some of such embodiments, the compositions described herein provide sustained release of a corticosteroid into the cochlea for at least 14 days. In some of such embodiments, the compositions described herein provide sustained release of a corticosteroid into the cochlea for at least 21 days. In some of such embodiments, the compositions described herein provide sustained release of a corticosteroid into the cochlea for at least 28 days. In some of such embodiments, the corticosteroid is dexamethasone, dexamethasone acetate, or any salt, polymorph, prodrug, complex thereof. In some of such embodiments, the corticosteroid is prednisolone, or any salt, polymorph, prodrug, complex thereof. In some of such embodiments, the corticosteroid is methylprednisolone, or any salt, polymorph, prodrug, complex thereof.

JNK inhibitors

In some embodiments of the methods described herein, the otic formulations comprise JNK inhibitors that are compatible with the formulations disclosed herein. Examples of JNK inhibitors include and are not limited to minocycline; SB-203580 (4-(4-Fluorophenyl)-2-(4-methylsulfinyl phenyl)-5-(4-pyridyl)1H-imidazole); PD 169316 (4-(4-Fluorophenyl)-2-(4-nitrophenyl)-5-(4-pyridyl)-1H-imidazole); SB 202190 (4-(4-Fluorophenyl)-2-(4-hydroxyphenyl)-5-(4-pyridyl)1H-imidazole); RWJ 67657 (4-[4-(4-fluorophenyl)-1-(3-phenylpropyl)-5-(4-pyridinyl)-1H-imidazol-2-yl]-3-butyn-1-ol); SB 220025 (5-(2-Amino-4-pyrimidinyl)-4-(4-fluorophenyl)-1-(4-piperidinlyl)imidazole); or combinations thereof. In some embodiments, the agent which antagonizes the MAPK/JNK signaling cascade is D-JNKI-1 ((D)-hJIP$_{175-157}$-DPro-DPro-(D)-HIV-TAT$_{57-48}$), AM-111 (Auris), SP600125 (anthra[1,9-cd]pyrazol-6(2H)-one), JNK Inhibitor I ((L)-HIV-TAT$_{48-57}$-PP-JBD$_{20}$), JNK Inhibitor III ((L)-HIV-TAT$_{47-57}$-gaba-c-Jun$\delta_{33-57}$), AS601245 (1,3-benzothiazol-2-yl(2-[[2-(3-pyridinyl)ethyl]amino]-4pyrimidinyl) acetonitrile), JNK Inhibitor VI ($H_2N$-RPKRPTTLNLF-$NH_2$), JNK Inhibitor VIII (N-(4-Amino-5-cyano-6-ethoxypyridin-2-yl)-2-(2,5-dimethoxyphenyl)acetamide), JNK Inhibitor IX (N-(3-Cyano-4,5,6,7-tetrahydro-1-benzothien-2-yl)-1-naphthamide), dicumarol (3,3'-Methylenebis(4-hydroxycoumarin)), SC-236 (4-[5-(4-chlorophenyl)-3-(trifluoromethyl)-1*H*-pyrazol-1-yl]benzene-sulfonamide), CEP-1347 (Cephalon), CEP-11004 (Cephalon); or combinations thereof.

JNK inhibitors that are not disclosed herein but which are useful in sustained release formulations and methods described herein are expressly included and intended within the scope of the methods presented.

In some embodiments, the compositions described herein provide release of a JNK inhibitor into the cochlea for a day. In other embodiments, the compositions described herein provide sustained release of a JNK inhibitor into the cochlea after a single administration. In some of such embodiments, the compositions described herein provide sustained release of a JNK inhibitor into the cochlea for at least 2 days. In some of such embodiments, the compositions described herein provide sustained release of a JNK inhibitor into the cochlea for at least 3 days. In some of such embodiments, the compositions described herein provide sustained release of a JNK inhibitor into the cochlea for at least 4 days. In some of such embodiments, the compositions described herein provide sustained release of a JNK inhibitor into the cochlea for at least 5 days. In some of such embodiments, the compositions described herein provide sustained release of a JNK inhibitor into the cochlea for at least 6 days. In some of such embodiments, the compositions described herein provide sustained release of a JNK inhibitor into the cochlea for at least 7 days. In some of such embodiments, the compositions described herein provide sustained release of a JNK inhibitor into the cochlea for at least 8 days. In some of such embodiments, the compositions described herein provide sustained release of a JNK inhibitor into the cochlea for at least 9 days. In some of such embodiments, a compositions described herein provide sustained release of a JNK inhibitor into the cochlea for at least 10 days. In some of such embodiments, the compositions described herein provide sustained release of a JNK inhibitor into the cochlea for at least 14 days. In some of such embodiments, the compositions described herein provide sustained release of a JNK inhibitor into the cochlea for at least 21 days. In some of such embodiments, the compositions described herein provide sustained release of a JNK inhibitor into the cochlea for at least 28 days. In some of such embodiments, the JNK inhibitor is SP600125, or any salt, polymorph, complex thereof.

Trophic factors

In some embodiments of the methods described herein, the otic formulations comprise trophic factors that are compatible with the formulations disclosed herein. Accordingly, some embodiments incorporate the use of trophic agents which promote the survival of neurons and otic hair cells, and/or the growth of neurons and otic hair cells. In some embodiments, the trophic agent which promotes the survival of otic hair cells is a growth factor. In some embodiments, the growth factor is a neurotroph. In some embodiments, the neurotroph is brain-derived neurotrophic factor (BDNF), ciliary neurotrophic factor (CNTF), glial cell-line derived neurotrophic factor (GDNF), neurotrophin-3, neurotrophin-4, and/or combinations thereof. In some embodiments, the growth factor is a fibroblast growth factor (FGF), an insulin-like

growth factor (IGF), an epidermal growth factor (EGF), a platlet-derived growth factor (PGF) and/or agonists thereof. In some embodiments, the growth factor is an agonist of the fibroblast growth factor (FGF) receptor, the insulin-like growth factor (IGF) receptor, the epidermal growth factor (EGF) receptor, and/or the platlet-derived growth factor. In some embodiments, the growth factor is hepatocyte growth factor.

In some embodiments, the trophic agent and/or neurotroph is BDNF. In some embodiments, the trophic agent and/or neurotroph is GDNF. In some embodiments, the neurotroph is neurotrophin-3 or

CNTF. In some embodiments, the trophic agent and/or growth factor is an epidermal growth factor (EGF). In some embodiments, the EGF is heregulin (HRG).

In some embodiments, the trophic agent and/or growth factor is an insulin-like growth factor (IGF). In some embodiments, the IGF is IGF-1. In some embodiments, the growth factor is hepatocyte growth factor (HGF).

Also contemplated for use in the otic formulations described herein are growth factors including Erythropoietin (EPO), Granulocyte-colony stimulating factor (G-CSF), Granulocyte-macrophage colony stimulating factor (GM-CSF), Growth differentiation factor-9 (GDF9), Insulin-like growth factor (IGF), Myostatin (GDF-8), Platelet-derived growth factor (PDGF), Thrombopoietin (TPO), Transforming growth factor alpha (TGF-$\alpha$), Transforming growth factor beta (TGF-$\beta$), Vascular endothelial growth factor (VEGF) or combinations thereof.

In some embodiments, the compositions described herein provide release of a trophic factor (e.g., IGF-1) into the cochlea for a day. In other embodiments, the compositions described herein provide sustained release of a trophic factor (e.g., IGF-1) into the cochlea after a single administration. In some of such embodiments, the compositions described herein provide sustained release of a trophic factor (e.g., IGF-1)into the cochlea for at least 2 days. In some of such embodiments, the compositions described herein provide sustained release of a trophic factor (e.g., IGF-1) into the cochlea for at least 3 days. In some of such embodiments, the compositions described herein provide sustained release of a trophic factor (e.g., IGF-1) into the cochlea for at least 4 days. In some of such embodiments, the compositions described herein provide sustained release of a trophic factor (e.g., IGF-1) into the cochlea for at least 5 days. In some of such embodiments, the compositions described herein provide sustained release of a trophic factor (e.g., IGF-1) into the cochlea for at least 6 days. In some of such embodiments, the compositions described herein provide sustained release of a trophic factor (e.g., IGF-1) into the cochlea for at least 7 days. In some of such embodiments, the compositions described herein provide sustained release of a trophic factor (e.g., IGF-1) into the cochlea for at least 8 days. In some of such embodiments, the compositions described herein provide sustained release of a trophic factor (e.g., IGF-1) into the cochlea for at least 9 days. In some of such embodiments, a compositions described herein provide sustained release of a trophic factor (e.g., IGF-1) into the cochlea for at least 10 days. In some of such embodiments, the compositions described herein provide sustained release of a trophic factor (e.g., IGF-1) into the cochlea for at least 14 days. In some of such embodiments, the compositions described herein provide sustained release of a trophic factor (e.g., IGF-1) into the cochlea for at least 21 days. In some of such embodiments, the compositions described herein provide sustained release of a trophic factor (e.g., IGF-1) into the cochlea for at least 28 days.

**Administration of otic compositions**

In some embodiments of the methods described herein, auris formulations described herein are administered intratympanically. In some embodiments, the otic compositions are administered at or near the round window membrane. In some embodiments, the otic compositions are administered in the vestibule of the ear and/or the ear canal and/or the middle ear. Localized administration in the ear reduces or eliminates systemic accumulation of the active agent.

In some embodiments of the methods provided herein, otic formulations are adminsitered at a suitable temperature (e.g., a temperature close to room temperature, e.g., about 20 °C) that avoids incidence of vertigo that is associated with administration of cold formulations (e.g., formulations having a temperature at time of administration of below about room temparature). Further, the formulations comprise polymers (e.g. thermoreversible polymers) that are biocompatible and/or otherwise non-toxic to the inner ear environment. In some embodiments, the gel polymer is biodegradable and/or bioeliminated (e.g., the copolymer is eliminated from the body by a biodegradation process, e.g., elimination in the urine, the feces or the like). The formulations are injectable liquids and gel upon contact with auditory surfaces.

In some embodiments, a composition disclosed herein (e.g., a gel formulation or a viscous fomulation comprising a multiparticulate corticosteroid or a multiparticulate JNK inhibitor) is administered to an individual in need thereof once prior to onset of treatment with an ototoxicity-inducing drug as described herein. In some embodiments, a composition disclosed herein (e.g., a gel formulation or a viscous fomulation comprising a multiparticulate corticosteroid or a multiparticulate JNK inhibitor) is administered to an individual in need thereof more than once prior to onset of treatment with an ototoxicity-inducing drug as described herein. In some embodiments, a composition disclosed herein (e.g., a gel formulation or a viscous fomulation comprising a multiparticulate corticosteroid or a multiparticulate JNK inhibitor) is administered to an individual in need thereof 24 hours prior to onset of treatment with an ototoxicity-inducing drug as described herein. In some embodiments, a composition disclosed herein (e.g., a gel formulation or a viscous fomulation

comprising a multiparticulate corticosteroid or a multiparticulate JNK inhibitor) is administered to an individual in need thereof 48 hours prior to onset of treatment with an ototoxicity-inducing drug as described herein. In some embodiments, a composition disclosed herein (e.g., a gel formulation or a viscous fomulation comprising a multiparticulate corticosteroid or a multiparticulate JNK inhibitor) is administered to an individual in need thereof 72 hours prior to onset of treatment with an ototoxicity-inducing drug as described herein. In some embodiments, a composition disclosed herein (e.g., a gel formulation or a viscous fomulation comprising a multiparticulate corticosteroid or a multiparticulate JNK inhibitor) is administered to an individual in need thereof 96 hours prior to onset of treatment with an ototoxicity-inducing drug as described herein. In some embodiments, a composition disclosed herein (e.g., a gel formulation or a viscous fomulation comprising a multiparticulate corticosteroid or a multiparticulate JNK inhibitor) is administered to an individual in need thereof 120 hours prior to onset of treatment with an ototoxicity-inducing drug as described herein. In some embodiments, a composition disclosed herein (e.g., a gel formulation or a viscous fomulation comprising a multiparticulate corticosteroid or a multiparticulate JNK inhibitor) is administered to an individual in need thereof 1 week prior to onset of treatment with an ototoxicity-inducing drug as described herein.

In some other embodiments, the methods described herein allow for administration of a composition (e.g., a gel formulation or a viscous fomulation comprising a multiparticulate corticosteroid or a multiparticulate JNK inhibitor) to an individual following previous treatment course(s) of the ototoxicity-inducing drug and aids in recovery of hearing.

In yet other embodiments, the methods described herein allow for prevention of hearing loss due to acoustic trauma and comprise administration of a composition comprising a JNK inhibitor to an individual in need thereof.

In specific embodiments, administration of a composition disclosed herein (e.g., a gel formulation or a viscous fomulation comprising a multiparticulate corticosteroid or a multiparticulate JNK inhibitor) prior to administration of a platinum-based chemotherapeutic agent (e.g., cisplatin, carboplatin, oxiplatin, bis-platinates) prevents onset of ototoxicity and/or hearing loss (i.e., the formulation has a protective effect when administered prophylactically). In specific embodiments, administration of a composition disclosed herein (e.g., a gel formulation or a viscous fomulation comprising a multiparticulate corticosteroid or a multiparticulate JNK inhibitor) prior to administration of a platinum-based chemotherapeutic agent (e.g., cisplatin, carboplatin, oxiplatin, bis-platinates) reduces severity of ototoxicity and/or hearing loss (i.e., the formulation has a protective effect when administered prophylactically).

In specific embodiments, administration of a composition disclosed herein (e.g., a gel formulation or a viscous fomulation comprising a multiparticulate corticosteroid or a multiparticulate JNK inhibitor) prior to administration of an aminoglycoside antibiotic (e.g., vancomycin, gentamicin) prevents onset of ototoxicity and/or hearing loss (i.e., the formulation has a protective effect when administered prophylactically). In specific embodiments, administration of a composition disclosed herein (e.g., a gel formulation or a viscous fomulation comprising a multiparticulate corticosteroid or a multiparticulate JNK inhibitor) prior to administration of an aminoglycoside antibiotic (e.g., vancomycin, gentamicin) reduces severity of ototoxicity and/or hearing loss (i.e., the formulation has a protective effect when administered prophylactically).

In specific embodiments, administration of a composition disclosed herein (e.g., a gel formulation comprising a multiparticulate corticosteroid (e.g., dexamethasone, dexamethasone acetate, prednisolone, methylprednisolone)) prior to administration of any ototoxicity-inducing agent (e.g., vincristine or any other agent described herein) prevents onset of ototoxicity and/or hearing loss (i.e., the formulation has a protective effect when administered prophylactically). In specific embodiments, administration of a composition disclosed herein (e.g., a gel formulation comprising a multiparticulate corticosteroid (e.g., dexamethasone, dexamethasone acetate, prednisolone, methylprednisolone)) prior to administration of any ototoxicity-inducing agent (e.g., vincristine or any other agent described herein) reduces severity of ototoxicity and/or hearing loss (i.e., the formulation has a protective effect when administered prophylactically).

In specific embodiments, administration of a composition disclosed herein (e.g., a thermoreversible gel formulation comprising a copolymer of polyoxyethylene and polyoxypropylene and a multiparticulate corticosteroid, (e.g., dexamethasone, dexamethasone acetate, prednisolone, methylprednisolone) or a multiparticulate JNK inhibitor (e.g., SP600125)) prior to exposure to acoustic trauma prevents onset of ototoxicity and/or hearing loss (i.e., the formulation has a protective effect when administered prophylactically). In specific embodiments, administration of a composition disclosed herein (e.g., a thermoreversible gel formulation comprising a copolymer of polyoxyethylene and polyoxypropylene and a multiparticulate corticosteroid, (e.g., dexamethasone, dexamethasone acetate, prednisolone, methylprednisolone) or a multiparticulate JNK inhibitor (e.g, SP600125)) prior to exposre to acoustic trauma reduces severity of ototoxicity and/or hearing loss (i.e., the formulation has a protective effect when administered prophylactically).

In some embodiments, a composition described herein (e.g., a thermoreversible gel formulation comprising a copolymer of polyoxyethylene and polyoxypropylene and a multiparticulate corticosteroid, (e.g., dexamethasone, dexamethasone acetate, prednisolone, methylprednisolone)) is administered prior to onset of therapy with an ototoxicity inducing drug (e.g., cisplatin, an aminoglycoside antibiotic or any other ototoxicity inducing drug described herein) and is also administered during treatment with the ototoxicity inducing drug. In some embodiments, a composition described herein (e.g., a thermoreversible gel formulation comprising a copolymer of polyoxyethylene and polyoxypropylene and a multiparticulate corticosteroid, (e.g., dexamethasone, dexamethasone acetate, prednisolone, methylprednisolone) or

a multiparticulate JNK inhibitor (e.g., SP600125)) is administered prior to exposure to acoustic trauma and is also administered during exposure to acoustic trauma.

The number of times a composition described herein is administered to an individual in need thereof depends on the discretion of a medical professional, the individuals's response to the formulation, and the ototoxicity-inducing drug treatment that the individual is undergoing, or the exposure to acoustic trauma.

In certain embodiments, patients require intermittent treatment and/or maintenance treatment on a long-term basis to avoid recurrence of symptoms.

**Otic compositions**

In some embodiments of the methods described herein, an auris formulation described herein (e.g., a gel formulation comprising a multiparticulate active agent) comprises between about 10%, 15%, 20%, 25%, 30%, 35%, 40%, 45%, 50%, or 55% and about 15%, 20%, 25%, 30%, 35%, 40%, 45%, or 50% of a gel-forming polymer In some embodiments of the methods described herein, an auris formulation described herein (e.g., a thermoreversible gel formulation comprising a copolymer of polyoxyethylene and polyoxypropylene and a corticosteroid) comprises between about 10%, 15%, 20%, 25%, 30%, 35%, 40%, 45%, 50%, or 55% and about 15%, 20%, 25%, 30%, 35%, 40%, 45%, or 50% of a thermoreversible polymer (e.g., a copolymer of polyoxyethylene and polyoxypropylene). In some embodiments, the resulting formulation is a thermoreversible gel, but it need not be thermoreversible; that is, depending on the amount of thermoreversible polymer, the resulting gel may be thermoreversible or not thermoreversible. The classification "thermoreversible polymer" refers to polymers that form thermoreversible gels in the range of about 15-42 degrees Celsius.

Polymers composed of polyoxypropylene and polyoxyethylene form gels when incorporated into aqueous solutions. These polymers have the ability to change from the liquid state to the gel state at temperatures close to body temperature, therefore allowing for preparation of useful formulations that are applied to the targeted structure(s). The liquid state-to-gel state phase transition (gelation temperature) is dependent on the polymer concentration, buffer concentration and the ingredients in the solution. In some embodiments, a thermoreversible gel suitable for compositions described herein is an aqueous gel comprising of a polymer of polyoxypropylene and polyoxyethylene.

Poloxamer (pluronic, Lutrol, Pluracare) is a synthetic block polymer of ethylene oxide and propylene oxide. Poloxamer 407 (F-127, P407) is a theroreversible polymer composed of polyoxyethylene-polyoxypropylene copolymers. Other poloxamers include 124, 188 (F-68 grade), 237 (F-87 grade), and 338 (F-108 grade). Aqueous solutions of poloxamers are stable in the presence of acids, alkalis, and metal ions. F-127 (or P407) is a commercially available polyoxyethylene-polyoxypropylene triblock copolymer, with an average molar mass of 13,000. The polymer can be further purified by suitable methods that will enhance gelation properties of the polymer. It contains approximately 70% ethylene oxide, which accounts for its hydrophilicity. It is one of the series of poloxamer ABA block copolymers, whose members share the chemical formula shown below.

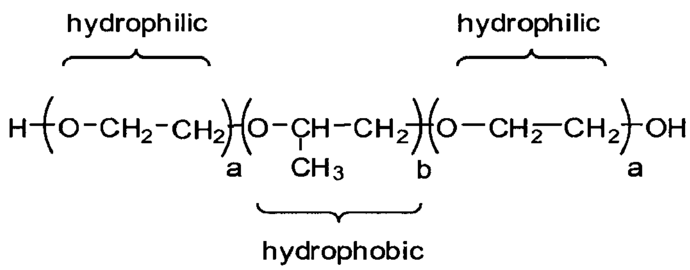


Poloxamers are available in several types, and with varying molecular weights ranging from about 2000 to about 15000. The □-hydro-□-hydroxypoly(oxyethylene)$_a$ poly(oxypropylene)$_b$poly(oxyethylene)$_a$ block copolymers comprise varying ratios of a and b as shown below:

| poloxamer | a | b |
|---|---|---|
| 124 | 12 | 20 |
| 188 | 80 | 27 |
| 237 | 64 | 37 |
| 338 | 141 | 44 |

(continued)

| poloxamer | a | b |
|---|---|---|
| 407 | 101 | 56 |

In certain embodiments, a thermoreversible gel formulation described herein comprises a poloxamer. In specific embodiments, a thermoreversible gel formulation described herein comprises P407. When placed in contact with auditory surfaces, such a gel preparation will form a semi-solid structure and a sustained release depot. Furthermore, poloxamers (e.g., P407) have good solubilizing capacity, low toxicity, and are biocompatible. In further embodiments, a formulation described herein is a viscous or thickened preparation. In yet further embodiments, a viscous or thickened preparation forms a gel upon contact with auditory surfaces.

In some embodiments of the methods described herein, an otic formulation described herein comprises between about 2.0% and about 80% of a gel-forming polymer (e.g., any polymer described herein) by weight of the composition. In some embodiments of the methods described herein, an otic formulation described herein comprises between about 2.0% and about 50% of a gel-forming polymer (e.g., any polymer described herein) by weight of the composition. In some embodiments of the methods described herein, an otic formulation described herein comprises between about 5.0% and about 30% of a gel-forming polymer (e.g., any polymer described herein) by weight of the composition.

In some embodiments of the methods described herein, an otic formulation described herein comprises between about 2.0% and about 50% of a thermoreversible polymer (e.g., polyoxyethylene-polyoxypropylene triblock copolymer) by weight of the composition. In some embodiments of the methods described herein, an otic formulation described herein comprises between about 2.0% and about 40% of a thermoreversible polymer (e.g., polyoxyethylene-polyoxypropylene triblock copolymer) by weight of the composition. In some embodiments of the methods described herein, an otic formulation described herein comprises between about 5.0% and about 30% of a thermoreversible polymer (e.g., polyoxyethylene-polyoxypropylene triblock copolymer) by weight of the composition. In some embodiments of the methods described herein, an otic formulation described herein comprises between about 10.0% and about 25% of a thermoreversible polymer (e.g., polyoxyethylene-polyoxypropylene triblock copolymer) by weight of the composition. In some embodiments of the methods described herein, an otic formulation described herein comprises between about 12.0% and about 25% of a thermoreversible polymer (e.g., polyoxyethylene-polyoxypropylene triblock copolymer) by weight of the composition. In some embodiments of the methods described herein, an otic formulation described herein comprises between about 14.0% and about 25% of a thermoreversible polymer (e.g., polyoxyethylene-polyoxypropylene triblock copolymer) by weight of the composition. In some embodiments, a formulation described herein comprises between about 14.5% and about 25% of a thermoreversible polymer (e.g., polyoxyethylene-polyoxypropylene triblock copolymer) by weight of the composition. In some embodiments, a formulation described herein comprises between about 15% and about 25% of a thermoreversible polymer (e.g., polyoxyethylene-polyoxypropylene triblock copolymer) by weight of the composition. In some embodiments, a formulation described herein comprises between about 10% and about 24% of a thermoreversible polymer (e.g., polyoxyethylene-polyoxypropylene triblock copolymer) by weight of the composition. In some embodiments, a formulation described herein comprises between about 12% and about 24% of a thermoreversible polymer (e.g., polyoxyethylene-polyoxypropylene triblock copolymer) by weight of the composition. In some embodiments, a formulation described herein comprises between about 15% and about 24% of a thermoreversible polymer (e.g., polyoxyethylene-polyoxypropylene triblock copolymer) by weight of the composition. In some embodiments, a formulation described herein comprises between about 10% and about 23% of a thermoreversible polymer (e.g., polyoxyethylene-polyoxypropylene triblock copolymer) by weight of the composition. In some embodiments, a formulation described herein comprises between about 12% and about 23% of a thermoreversible polymer (e.g., polyoxyethylene-polyoxypropylene triblock copolymer) by weight of the composition. In some embodiments, a formulation described herein comprises between about 15% and about 23% of a thermoreversible polymer (e.g., polyoxyethylene-polyoxypropylene triblock copolymer) by weight of the composition. In some embodiments, a formulation described herein comprises between about 10% and about 22% of a thermoreversible polymer (e.g., polyoxyethylene-polyoxypropylene triblock copolymer) by weight of the composition. In some embodiments, a formulation described herein comprises between about 12% and about 22% of a thermoreversible polymer (e.g., polyoxyethylene-polyoxypropylene triblock copolymer) by weight of the composition. In some embodiments, a formulation described herein comprises between about 15% and about 22% of a thermoreversible polymer (e.g., polyoxyethylene-polyoxypropylene triblock copolymer) by weight of the composition.

In some embodiments, a formulation described herein comprises between about 10% and about 21% of a thermoreversible polymer (e.g., polyoxyethylene-polyoxypropylene triblock copolymer) by weight of the composition. In some embodiments, a formulation described herein comprises between about 12% and about 21% of a thermoreversible polymer (e.g., polyoxyethylene-polyoxypropylene triblock copolymer) by weight of the composition. In some embodiments, a formulation described herein comprises between about 15% and about 21% of a thermoreversible polymer

(e.g., polyoxyethylene-polyoxypropylene triblock copolymer) by weight of the composition.

In some embodiments of the methods described herein, an otic formulation described herein comprises between about 10.0% and about 20% of a thermoreversible polymer (e.g., polyoxyethylene-polyoxypropylene triblock copolymer) by weight of the composition. In some embodiments of the methods described herein, an otic formulation described herein comprises between about 12.0% and about 20% of a thermoreversible polymer (e.g., polyoxyethylene-polyoxypropylene triblock copolymer) by weight of the composition. In some embodiments of the methods described herein, an otic formulation described herein comprises between about 15.0% and about 20% of a thermoreversible polymer (e.g., polyoxyethylene-polyoxypropylene triblock copolymer) by weight of the composition.

In some embodiments of the methods described herein, an otic formulation described herein comprises between about 10.0% and about 18% of a thermoreversible polymer (e.g., polyoxyethylene-polyoxypropylene triblock copolymer) by weight of the composition. In some embodiments of the methods described herein, an otic formulation described herein comprises between about 12.0% and about 18% of a thermoreversible polymer (e.g., polyoxyethylene-polyoxypropylene triblock copolymer) by weight of the composition. In some embodiments of the methods described herein, an otic formulation described herein comprises between about 15.0% and about 18% of a thermoreversible polymer (e.g., polyoxyethylene-polyoxypropylene triblock copolymer) by weight of the composition.

In some embodiments, a formulation described herein comprises between about 16% and about 21% of a thermoreversible polymer (e.g., polyoxyethylene-polyoxypropylene triblock copolymer) by weight of the composition. In some embodiments of the methods described herein, an otic formulation described herein comprises between about 16.0% and about 20% of a thermoreversible polymer (e.g., polyoxyethylene-polyoxypropylene triblock copolymer) by weight of the composition. In some of such embodiments, a thermoreversible polymer comprises a copolymer of polyoxyethylene and polyoxypropylene. In any of the embodiments described above and herein, the thermoreversible polymer is a poloxamer. In some of such embodiments, the poloxamer is P407 (also known as Lutrol F127, Pluracare F127, F-127, Pol-407, or Pluronic-127). In any of the embodiments described above and herein, the thermoreversible polymer is a fractionated polymer as described herein.

Also contemplated within the scope of methods described herein are otic compositions comprising other viscosity enhancing polymers. "Viscosity enhancing polymers" are polymers that increase viscosity of a formulation described herein so that the formulation forms a thickened liquid upon administration. In some embodiments, viscosity enhancing polymers are gel-forming polymers. In some embodiments, a viscosity enhancing polymer is a thermosensitive polymer. In some embodiments, a thermosensitive polymer is not a thermoreversible polymer. In other embodiments, a thermosensitive polymer is a thermoreversible polymer.

Suitable viscosity-enhancing polymers include and are not limited to, hydrogels (e.g., chitosan), gelatin, hyaluronic acid (including and not limited to Hyalastine®, Hyalectin®, Hyaloftil® and/or partial esters and/or salts thereof (e.g., barium salt of hyaluronic acid, or any other salt of hyaluronic acid described in WO/1998/017285, salts described therein are incorporated herein by reference)), acrylic acid based polymers (e.g., Carbopol®), MedGel®, cellulose based polymers (e.g., carboxymethylcellulose), polymers comprising polyoxyethylene-polyoxypropylene triblock copolymers, poloxamers, or any other such polymer described herein. In some of such embodiments, the resulting formulation is a thermosensitive gel, but it need not be thermoreversible; that is, depending on the amount of thermosensitive polymer, the resulting gel may be thermoreversible or not thermoreversible. In some embodiments, an otic composition described herein comprises hyaluronic acid as a viscosity enhancing polymer.

In alternative embodiments, the gel-forming polymers are co-polymers comprising lactic acid and glycolic acid monomers. PLGA or poly(lactic-co-glycolic acid) is a co-polymer of two different monomers, glycolic acid and lactic acid. Depending on the ratio of lactide to glycolide used for the polymerization, different forms of PLGA are obtained (e.g. PLGA 75:25 which is a copolymer whose composition is 75% lactic acid and 25% glycolic acid). In some embodiments, an otic composition described herein comprises PLGA as a viscosity enhancing polymer.

In an alternative embodiment, the thermosensitive gel comprises PEG-PLGA-PEG triblock copolymer (Jeong etal, Nature (1997), 388:860-2; Jeong etal, J. Control. Release (2000), 63:155-63; Jeong etal, Adv. Drug Delivery Rev. (2002), 54:37-51). The polymer exhibits sol-gel behavior over a concentration of about 5% w/w to about 40% w/w. Depending on the properties desired, the lactide/glycolide molar ratio in the PLGA copolymer ranges from about 1:1 to about 20:1. The resulting coploymers are soluble in water and form a free-flowing liquid at room temperature, but form a gel at body temperature.

ReGel® is a tradename of MacroMed Incorporated for a class of low molecular weight, biodegradable block copolymers having reverse thermal gelation properties as described in U.S. Pat. Nos. 6,004,573, 6,117949, 6,201,072, and 6,287,588. It also includes biodegradable polymeric drug carriers disclosed in pending U.S. patent application Ser. Nos. 09/906,041, 09/559,799 and 10/919,603. The biodegradable drug carrier comprises ABA-type or BAB-type triblock copolymers or mixtures thereof, wherein the A-blocks are relatively hydrophobic and comprise biodegradable polyesters or poly(orthoester)s, and the B-blocks are relatively hydrophilic and comprise polyethylene glycol (PEG), said copolymers having a hydrophobic content of between 50.1 to 83% by weight and a hydrophilic content of between 17 to 49.9% by weight, and an overall block copolymer molecular weight of between 2000 and 8000 Daltons.

In some embodiments, other thermosensitive polymers are useful depending upon the particular active agent, other pharmaceutical agent or excipients/additives used, and as such are considered to fall within the scope of the present disclosure. For example, other commercially-available glycerin-based gels, glycerin-derived compounds, conjugated, or crosslinked gels, matrices, hydrogels, and polymers, as well as gelatins and their derivatives, alginates, and alginate-based gels, and even various native and synthetic hydrogel and hydrogel-derived compounds are all expected to be useful in the pharmaceutical formulations described herein. In some embodiments, bioacceptable gels include, but are not limited to, alginate hydrogels SAF®-Gel (ConvaTec, Princeton, N.J.), Duoderm® Hydroactive Gel (ConvaTec), Nu-gel ®(Johnson & Johnson Medical, Arlington, Tex.); Carrasyn®(V) Acemannan Hydrogel (Carrington Laboratories, Inc., Irving, Tex.); glycerin gels Elta® Hydrogel (Swiss-American Products, Inc., Dallas, Tex.), K-Y® Sterile (Johnson & Johnson), gelatin hydrogels, chitosan, silicon-base gels (e.g., Medgel®) or the like. Other thermosensitive and/or bioacceptable gels suitable for compositions described herein include acrylic acid-based polymers (e.g., Carbopol®), cellulose based polymers (e.g., hydroxypropylmethyl cellulose, carboxymethyl cellulose, or the like), alkyl aryl polyether alcohol-based polymer (e.g., Tyloxapol®), or the like.

In some embodiments, for any intratympanic composition described above and herein, the composition comprises a gel. In some embodiments, any intratympanic formulation described above and herein is a liquid at time of administration and gels in situ in the ear and/or forms a thickened preparation in the ear. In some embodiments, the liquid to gel transition of the formulation is thermoreversible. In some embodiments, the intratympanic formulation comprises a rheopectic material (e.g., the material becomes more viscous when shaken or stressed). For example, when a rheopectic formulation is injected into a patient's ear via syringe under normal pressure, such as stress or pressure exerted with a thumb by an otolaryngologist, the liquid formulation transitions to a gel or a thickened formulation. In some embodiments, the intratympanic compositions described herein are non-Newtonian fluids, i.e., the relationship between shear stress and velocity gradient is not linear. For example, in one embodiment, the viscosity of an intratympanic gel formulation described herein increases with the rate of shear. In some embodiments, the intratympanic formulation comprises a material which gels by cross-linking. In some embodiments, any intratympanic composition described above and herein has a syringable viscosity. In other words, the compositions described above and herein are injectable via a syringe with a narrow gauge needle (e.g., a needle of between about 14 - 34 gauge, or a needle of between about 18-31 gauge, or a needle of between about 22-31 gauge) using normal pressure (e.g., the pressure exerted by the thumb when a surgeon injects the formulation intratympanically).

In some embodiments, for any intratympanic composition described above and herein, the composition has a thickened viscosity (e.g., gel viscosity) of between about 10 cP and about 1000,000 cP. In some embodiments, for any intratympanic composition described above and herein, the composition has a thickened viscosity of between about 10 cP and about 500,000 cP. In some embodiments, for any intratympanic composition described above and herein, the composition has a thickened viscosity of between about 10 cP and about 250,000 cP. In some embodiments, for any intratympanic composition described above and herein, the composition has a thickened viscosity of between about 10 cP and about 100,000 cP. In some embodiments, for any intratympanic composition described above and herein, the composition has a thickened viscosity of between about 10 cP and about 10,000 cP. In some embodiments, for any intratympanic composition described above and herein, the composition has a thickened viscosity of between about 10 cP and about 5,000 cP. In some embodiments, for any intratympanic composition described above and herein, the composition has a thickened viscosity of between about 25 cP and about 1,000 cP.

In some embodiments, for any intratympanic composition described above and herein, the composition is a thickened or viscous composition having a viscosity of at least 30 cP. In some embodiments, for any intratympanic composition described above and herein, the composition is a thickened or viscous composition having a viscosity of at least 50 cP. In some embodiments, for any intratympanic composition described above and herein, the composition is a thickened or viscous composition having a viscosity of at least 100 cP. In some embodiments, for any intratympanic composition described above and herein, the composition is a thickened or viscous composition having a viscosity of at least 200 cP. In some embodiments, for any intratympanic composition described above and herein, the composition is a thickened or viscous composition having a viscosity of at least 500 cP. In some embodiments, for any intratympanic composition described above and herein, the composition is a thickened or viscous composition having a viscosity of at least 1000 cP. In some embodiments, for any intratympanic composition described above and herein, the composition is a thickened or viscous composition having a viscosity of at least 5000 cP. In some embodiments, for any intratympanic composition described above and herein, the composition is a thickened or viscous composition having a viscosity of at least 10,000 cP. In some embodiments, for any intratympanic composition described above and herein, the composition is a thickened or viscous composition having a viscosity of at least 25,000 cP. In some embodiments, for any intratympanic composition described above and herein, the composition is a thickened or viscous composition having a viscosity of at least 50,000 cP. In some embodiments, for any intratympanic composition described above and herein, the composition is a thickened or viscous composition having a viscosity of at least 100,000 cP. In some embodiments, for any intratympanic composition described above and herein, the composition is a thickened or viscous composition having a viscosity of at least 250,000 cP. In some embodiments, for any intratympanic composition described above and herein, the

composition is a thickened or viscous composition having a viscosity of at least 500,000 cP. In some embodiments, for any intratympanic composition described above and herein, the composition is a thickened or viscous composition having a viscosity of at least 1000,000 cP.

**Purification poly(oxyethylene)/poly(oxypropylene) triblock polymers**

Also contemplated within the scope of embodiments presented herein is the use of purified theremoreversible polymers. As used herein, a "purified" thermoreversible polymer is a commercially purchased thermoreversible polymer that is subjected to further steps prior to preparation of formulations described herein. A purified thermoreversible polymer has lower polydispersity (i.e., a narrower distribution of molecular weights amongst the individual polymer chains therein) and/or lower ethylene content and/or less unsaturation and/or weight% oxyethylene values compared to a commercially available sample of the same polymer. Purification is carried out using any suitable technique including and not limited to fractionation, chromatography, washing and/or decantation, purification using supercritical fluid (*See,* for example, U.S. Patent Appl. Pub. No. 2008/0269449, disclosure of purification of polymers by use of supercritical fluid described therein is incorporated herein by reference), reverse precipitation (*See,* for example, U.S. Patent No. 7,148,320, disclosure of reverse precipitation described therein is incorporated herein by reference), salt extraction and liquid phase separation (*See* for example, U.S. Patent No. 5,800,711, disclosure of poloxamer purification described therein is incorporated herein by reference), or the like. Other processes for purification and/or fractionation of polymers are described in, for example, US 6,977,045 and US 6,761,824 which processes described therein are incorporated herein by reference

By way of example, in some embodiments, purified poloxamer 407 is fractionated P407 having a lower polydispersity index compared to a commercially purchased batch of P407 grade NF from BASF. By way of example, the commercially purchased P407 has a polydispersity index of about 1.2. In some embodiments, the polydispersity index of fractionated P407 as described herein is between about 1 and about 1.15. In other embodiments, the polydispersity index of fractionated P407 as described herein is between about 1 and about 1.1. In yet other embodiments, the polydispersity index of fractionated P407 as described herein is between about 1 and about 1.05. As used herein, the calculated polydispersity index (PDI) is the weight average molecular weight divided by the number average molecular weight of polymeric chains ($M_w/M_n$). It indicates the distribution of individual molecular masses in a batch of polymers.

The purification of poloxamers is based on the removal of low molecular weight components (e.g., oligomers, unreacted material and/or other unwanted impurities that are produced during manufacturing or storage) and/or large molecular weight components (components from unwanted polymer-polymer reactions). The resulting purified product has a narrower PDI with approximately the same molecular weight as the original material. In some embodiments, a purified poloxamer has better gelling characteristics (e.g., a lower Tgel for the same % poloxamer concentration while providing a higher viscosity in the gel state).

As used herein, a purified thermosensitive polymer has low polydispersity (i.e., a narrow distribution of molecular weights amongst the individual polymer chains therein). For example, commercially available poloxamers contain certain impurities such as poly(oxyethylene) homopolymer and poly(oxyethylene)/poly(oxypropylene) diblock polymers due to the nature of the manner in which they are produced. The relative amounts of these byproducts increase as the molecular weights of the component blocks increase. In some instances, in commercially available poloxamer 407, byproducts may constitute from about 15 to about 50% by weight of the polymer depending upon the manufacturer, thereby resulting in high polydispersity. Example 6 illustrates a procedure for fractionation of P407 that reduces polydispersity in commercially available P407.

In some embodiments, super critical fluid extraction technique is used to fractionate polyoxyalkylene block copolymers. *See,* U.S. Pat. No. 5,567,859, the disclosure for fractionation of polymers described therein is incorporated herein by reference. In this technique, lower molecular weight fractions in commercially purchased polymer are removed in a stream of $CO_2$ maintained at a pressure of 2200 pounds per square inch (psi) and a temperature of 40 °C, thereby providing purified polymer having low polydispersity.

In some embodiments, gel permeation chromoatography allows for isolation of fractions of polymers. *See,* European Patent Application WO 92/16484; the use of gel permeation chromatography to isolate a fraction of poloxamer having low polydispersity and saturation described therein is incorporated herein by reference.

In some embodiments, one or more of the blocks is purified prior to manufacture of the copolymer. By way of example, purifying either the polyoxypropylene center block during synthesis of the copolymer, or the copolymer product itself (*See,* U.S. Pat. Nos. 5,523,492, and 5,696,298, incorporated herein by reference for such disclosure) allows for manufacture of purified poloxamers.

In some embodiments, fractionation of polyoxyalkylene block copolymers is acheived by batchwise removal of low molecular weight species using a salt extraction and liquid phase separation technique (*See,* U.S. Pat. No. 5,800,711, which process of purification of polymers described therein is incorporated herein by reference). Such fractionation produces polyoxyalkylene block copolymers (e.g., poloxamer 407, poloxamer 188 or the like) having improved physical characteristics including increased gel strength, decreased polydispersity, higher average molecular weight, decreased

gelling concentration and/or extended gel dissolution profiles compared to commercially available poloxamers (e.g., P407 NF grade from BASF). Other processes for purification and/or fractionation of polymers are described in, for example, US 6,977,045 and US 6,761,824 which processes are incorporated herein by reference.

In some instances, low molecular weight contaminants of polymers (e.g., poloxamers) cause deleterious side effects in vivo; the use of purified poloxamers in pharmaceutical formulations described herein reduces such in vivo side effects.

Accordingly, also contemplated within the scope of embodiments presented herein are formulations comprising purified poly(oxyethylene)/poly(oxypropylene) triblock polymers that are substantially free of the poly(oxyethylene) homopolymers and/or poly(oxypropylene)/poly(oxyethylene) diblock byproducts, thereby narrowing the molecular weight distribution of block copolymers, (i.e., providing low polydispersity). In some embodiments, such purified poly(oxyethylene)/poly(oxypropylene) triblock polymers (e.g., fractionated poloxamers) allow for formulation of active compositions that comprise lower concentrations of the poly(oxyethylene)/poly(oxypropylene) triblock polymers compared to active compositions that comprise non-fractionated poly(oxyethylene)/poly(oxypropylene) triblock polymers.

**pH and Practical Osmolarity**

In some embodiments of the methods described herein, a pharmaceutical formulation disclosed herein is formulated to provide an ionic balance that is compatible biological fluids (e.g., endolymph and/or perilymph in an inner ear environment).

As used herein, "practical osmolarity/osmolality" or "deliverable osmolarity/osmolality" means the osmolarity/osmolality of a formulation as determined by measuring the osmolarity/osmolality of the active agent and all excipients except the thermosensitive polymer agent (e.g., polyoxyethylene-polyooxypropylene copolymers, or the like). The practical osmolarity of a formulation disclosed herein is measured by any suitable method, e.g., a freezing point depression method as described in Viegas et. al., Int. J. Pharm., 1998, 160, 157-162. In some instances, the practical osmolarity of a formulation disclosed herein is measured by vapor pressure osmometry (e.g., vapor pressure depression method) that allows for determination of the osmolarity of a formulation at higher temperatures. In some instances, vapor pressure depression method allows for determination of the osmolarity of a formulation comprising a a thermosensitive polymer at a higher temperature such as for example the gelation temperature of the thermosensitive polymer.

In some embodiments, the osmolarity at a target site of action (e.g., the perilymph in the inner ear,) is about the same as the practical osmolarity (i.e., osmolarity of materials that cross or penetrate the round window membrane in the ear) of a formulation described herein.

The practical osmolality of an pharmaceutical formulation disclosed herein is from about 100 mOsm/kg to about 1000 mOsm/kg, from about 200 mOsm/kg to about 800 mOsm/kg, from about 250 mOsm/kg to about 500 mOsm/kg, or from about 250 mOsm/kg to about 320 mOsm/kg, or from about 250 mOsm/kg to about 350 mOsm/kg or from about 280 mOsm/kg to about 320 mOsm/kg. In some embodiments, a formulation described herein has a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 250 mOsm/L to about 320 mOsm/L, or about 280 mOsm/L to about 320 mOsm/L. In some embodiments, the practical osmolality is estimated as an additive combination of buffer osmolality and the osmolality of the supernatant of the gelled poloxamer in water.

In some embodiments, useful formulations also include one or more pH adjusting agents or buffering agents. Suitable pH adjusting agents or buffers include, but are not limited to acetate, bicarbonate, ammonium chloride, citrate, phosphate, pharmaceutically acceptable salts thereof and combinations or mixtures thereof. In certain embodiments of the present disclosure, the amount of buffer included in the gel formulations are an amount such that the pH of the gel formulation does not interfere with the body's natural buffering system and/or the osmolarity of physiological fluids. In some embodiments, the pH of a formulation described herein is between about 3.0, 3.5, 4.0, 4.5, 5.0, 5.5, 6.0, 6.5, or 7.0 and about 7.0, 7.5, 8.0, 8.5, 9.0, 9.5, 10.0, 10.5, 11.0, 11.5, or 12.0. In some embodiments, the pH of a formulation described herein is between about 3.0 and about 12.0. In some embodiments, the pH of a formulation described herein is between about 4.5 and about 10.0. In some embodiments, the pH of a formulation described herein is between about 3.5 and about 9.0. In some embodiments, the pH of a formulation described herein is between about 3.5 and about 8.5. In some embodiments, the pH of a formulation described herein is between about 5.5 and about 8.0. In some embodiments, the pH of a formulation described herein is between about 6.5 and about 8.0. In some embodiments, the pH of a formulation described herein is between about 7.0 and about 7.8. In some embodiments, the pH of a formulation described herein is between about 7.0 and about 7.6. In some embodiments, the pH of a formulation described herein is between about 7.0 and about 7.4. In some embodiments, the pH of a formulation described herein is between about 7.4 and about 7.8.

In some embodiments, the formulations described herein have a concentration of active pharmaceutical ingredient between about 1 μM and about 10 μM, between about 1 mM and about 100 mM, between about 0.1 mM and about 100 mM, betwen about 0.1 mM and about 100 nM. In some embodiments, the formulations described herein have

a concentration of active pharmaceutical ingredient between about 0.001% - about 60%, between about 0.001% - about 40%, between about 0.01% - about 20%, between about 0.01% - about 10%, between about 0.01% - about 7.5%, between about 0.01% - 6%, between about 0.01 - 5%, between about 0.1% - about 40%, between about 0.1% - about 30%, between about 0.1% - about 20%, between about 0.1 - about 10%, or between about 0.1 - about 6% of the active ingredient by weight of the formulation. In some embodiments, formulations described herein have a concentration of active pharmaceutical agent between about 1% - about 40%, between about 5% - about 40%, between about 10% - about 40%, betwen about 15% - about 40%, between about 10% - about 30%, between about 10% - 20%, between about 15% - about 25%, or between about 20% - 30%, of the active ingredient by weight of the formulation. In some embodiments, the formulations described herein have a concentration of active pharmaceutical ingredient between about 1 μg/mL and about 500 μg/mL, between about 1 μg/mL and about 250 μg/mL, between about 1 μg and about 100 μg/mL, between about 1 μg/mL and about 50 μg/mL, or between about 1 μg/mL and about 20 μg/mL of the active agent in the formulation. In some embodiments, the formulations described herein have a concentration of active pharmaceutical ingredient between about 1 mg/mL and about 500 mg/mL, between about 1 mg/mL and about 400 mg/mL, between about 1 mg/mL and about 350 mg/mL, between about 1 mg/mL and about 250 mg/mL, between about 1 mg/mL and about 200 mg/mL, between about 1 mg/mL and about 100 mg/mL, between about 1 mg/mL and about 50 mg/mL, or between about 1 mg/mL and about 25 mg/mL of the active agent in the formulation.

**General Methods of Sterilization**

Provided herein are otic compositions which prevent or reverse or lessen the severity of otic conditions described herein. Further provided herein are methods comprising the administration of said otic compositions. In some embodiments, the compositions or devices are sterilized. Included within the embodiments disclosed herein are means and processes for sterilization of a pharmaceutical composition or device disclosed herein for use in humans. The goal is to provide a safe pharmaceutical product, relatively free of infection causing micro-organisms. The U. S. Food and Drug Administration has provided regulatory guidance in the publication "Guidance for Industry: Sterile Drug Products Produced by Aseptic Processing" available at: http://www.fda.gov/cder/guidance/5882fnl.htm, which is incorporated herein by reference in its entirety.

As used herein, sterilization means a process used to destroy or remove microorganisms that are present in a product or packaging. Any suitable method available for sterilization of objects and compositions is used. Available methods for the inactivation of microorganisms include, but are not limited to, the application of extreme heat, lethal chemicals, or gamma radiation. In some embodiments is a process for the preparation of an otic therapeutic formulation comprising subjecting the formulation to a sterilization method selected from heat sterilization, chemical sterilization, radiation sterilization or filtration sterilization. The method used depends largely upon the nature of the device or composition to be sterilized. Detailed descriptions of many methods of sterilization are given in Chapter 40 of Remington: The Science and Practice of Pharmacy published by Lippincott, Williams & Wilkins, and is incorporated by reference with respect to this subject matter.

*Sterilization by Heat*

Many methods are available for sterilization by the application of extreme heat. One method is through the use of a saturated steam autoclave. In this method, saturated steam at a temperature of at least 121 °C is allowed to contact the object to be sterilized. The transfer of heat is either directly to the microorganism, in the case of an object to be sterilized, or indirectly to the microorganism by heating the bulk of an aqueous solution to be sterilized. This method is widely practiced as it allows flexibility, safety and economy in the sterilization process.

Dry heat sterilization is a method which is used to kill microorganisms and perform depyrogenation at elevated temperatures. This process takes place in an apparatus suitable for heating HEPA-filtered microorganism-free air to temperatures of at least 130-180 °C for the sterilization process and to temperatures of at least 230-250 °C for the depyrogenation process. Water to reconstitute concentrated or powdered formulations is also sterilized by autoclave. In some embodiments, the formulations described herein comprise micronized antimicrobial agents (e.g., micronized demamethasone powder) that are sterilized by dry heating, e.g., heating for about 7 - 11 hours at internal powder temperatures of 130-140 °C, or for 1-2 hours at interrnal tempearatures of 150-180 °C.

*Chemical Sterilization*

Chemical sterilization methods are an alternative for products that do not withstand the extremes of heat sterilization. In this method, a variety of gases and vapors with germicidal properties, such as ethylene oxide, chlorine dioxide, formaldehyde or ozone are used as the anti-apoptotic agents. The germicidal activity of ethylene oxide, for example, arises from its ability to serve as a reactive alkylating agent. Thus, the sterilization process requires the ethylene

oxide vapors to make direct contact with the product to be sterilized.

*Radiation Sterilization*

One advantage of radiation sterilization is the ability to sterilize many types of products without heat degradation or other damage. The radiation commonly employed is beta radiation or alternatively, gamma radiation from a $^{60}Co$ source. The penetrating ability of gamma radiation allows its use in the sterilization of many product types, including solutions, compositions and heterogeneous mixtures. The germicidal effects of irradiation arise from the interaction of gamma radiation with biological macromolecules. This interaction generates charged species and free radicals. Subsequent chemical reactions, such as rearrangements and cross-linking processes, result in the loss of normal function for these biological macromolecules. The formulations described herein are also optionally sterilized using beta irradiation.

*Filtration*

Filtration sterilization is a method used to remove but not destroy microorganisms from solutions. Membrane filters are used to filter heat-sensitive solutions. Such filters are thin, strong, homogenous polymers of mixed cellulosic esters (MCE), polyvinylidene fluoride (PVF; also known as PVDF), or polytetrafluoroethylene (PTFE) and have pore sizes ranging from 0.1 to 0.22 □m. Solutions of various characteristics are optionally filtered using different filter membranes. For example, PVF and PTFE membranes are well suited to filtering organic solvents while aqueous solutions are filtered through PVF or MCE membranes. Filter apparatus are available for use on many scales ranging from the single point-of-use disposable filter attached to a syringe up to commercial scale filters for use in manufacturing plants. The membrane filters are sterilized by autoclave or chemical sterilization. Validation of membrane filtration systems is performed following standardized protocols (Microbiological Evaluation of Filters for Sterilizing Liquids, Vol 4, No. 3. Washington, D.C: Health Industry Manufacturers Association, 1981) and involve challenging the membrane filter with a known quantity (ca. $10^7/cm^2$) of unusually small microorganisms, such as Brevundimonas diminuta (ATCC 19146).

Pharmaceutical compositions are optionally sterilized by passing through membrane filters. Formulations comprising nanoparticles (U.S. Pat No. 6,139,870) or multilamellar vesicles (Richard et al., International Journal of Pharmaceutics (2006), 312(1-2):144-50) are amenable to sterilization by filtration through 0.22 μm filters without destroying their organized structure.

In some embodiments, the methods disclosed herein comprise sterilizing the formulation (or components thereof) by means of filtration sterilization. In another embodiment the auris-acceptable otic therapeutic agent formulation comprises multiparticulates where the formulation is suitable for filtration sterilization. In a further embodiment said particle formulation comprises particles of less than one micron in size, of less than 500 nm in size, less than 300 nm in size, of less than 200 nm in size, or of less than 100 nm in size, or a combination thereof. In another embodiment the auris-acceptable formulation comprises a particle formulation wherein the sterility of the particle is ensured by sterile filtration of the precursor component solutions. In another embodiment the auris-acceptable formulation comprises a particle formulation wherein the sterility of the particle formulation is ensured by low temperature sterile filtration. In a further embodiment, low temperature sterile filtration is carried out at a temperature between 0 and 30 °C, between 0 and 20 °C, between 0 and 10 °C, between 10 and 20 °C, or between 20 and 30 °C.

In another embodiment is a process for the preparation of an auris-acceptable multiparticulate formulation comprising: filtering the aqueous solution containing the particle formulation at low temperature through a sterilization filter; lyophilizing the sterile solution; and reconstituting the particle formulation with sterile water prior to administration. In some embodiments, a formulation described herein is manufactured as a suspension in a single vial formulation containing the multiparticulate (e.g., micronized) active pharmaceutical ingredient. A single vial formulation is prepared by aseptically mixing a sterile polymer solution (e.g., a poloxamer solution) with sterile micronized active ingredient (e.g., dexamethasone, SP600125 and the like) and transferring the formulation to sterile pharmaceutical containers. In some embodiments, a single vial containing a formulation described herein as a suspension is resuspended before dispensing and/or administration. By way of example, a polymer solution may be sterile filtered and a multiparticulate active agent (e.g., dexamethasone) is separately heat sterilized. The multiparticulate active agent is then aseptically suspended in the polymer solution to obtain the final sterile intratympanic composition.

In specific embodiments, filtration and/or filling procedures are carried out at about 5°C below the gel temperature (Tgel) of a formulation described herein and with viscosity below a theoretical value of 100cP to allow for filtration in a reasonable time using a peristaltic pump.

In another embodiment the auris-acceptable otic therapeutic agent formulation comprises a nanoparticle formulation wherein the nanoparticle formulation is suitable for filtration sterilization. In a further embodiment the nanoparticle formulation comprises nanoparticles of less than 300 nm in size, of less than 200 nm in size, or of less than 100 nm in size. In another embodiment the auris-acceptable formulation comprises a microsphere formulation wherein the sterility of the microsphere is ensured by sterile filtration of the precursor organic solution and aqueous solutions. In another

embodiment the auris-acceptable formulation comprises a gel formulation wherein the sterility of the gel formulation is ensured by low temperature sterile filtration. In a further embodiment, the low temperature sterile filtration occurs at a temperature between 0 and 30 °C, or between 0 and 20 °C, or between 0 and 10 °C, or between 10 and 20 °C, or between 20 and 30 °C. In another embodiment is a process for the preparation of an auris-acceptable gel formulation comprising: filtering the aqueous solution containing the gel components at low temperature through a sterilization filter; lyophilizing the sterile solution; and reconstituting the gel formulation with sterile water prior to administration.

In certain embodiments, the active ingredients are dissolved in a suitable vehicle (e.g. a buffer) and sterilized separately (e.g. by heat treatment, filtration, gamma radiation). In some instances, the active ingredients are sterilized separately in a dry state. In some instances, the active ingredients are sterilized as a suspension or as a colloidal suspension. The remaining excipients (e.g., fluid gel components present in auris formulations) are sterilized in a separate step by a suitable method (e.g. filtration and/or irradiation of a cooled mixture of excipients); the two solutions that are separately sterilized are then mixed aseptically to provide a final auris formulation. In some instances, the final aseptic mixing is performed just prior to administration of a formulation described herein.

In some instances, conventionally used methods of sterilization (e.g., heat treatment (e.g., in an autoclave), gamma irradiation, filtration) lead to irreversible degradation of polymeric components (e.g., thermosetting, gelling or mucoadhesive polymer components) and/or the active agent in the formulation. In some instances, sterilization of an auris formulation by filtration through membranes (e.g., 0.2 μM membranes) is not possible if the formulation comprises thixotropic polymers that gel during the process of filtration.

Accordingly, provided herein are methods for sterilization of auris formulations that prevent degradation of polymeric components (e.g., thermosetting and/or gelling and/or mucoadhesive polymer components) and/or the active agent during the process of sterilization. In some embodiments, degradation of the active agent (e.g., any therapeutic otic agent described herein) is reduced or eliminated through the use of specific pH ranges for buffer components and specific proportions of gelling agents in the formulations. In some embodiments, the choice of an appropriate gellling agent and/or thermosetting polymer allows for sterilization of formulations described herein by filtration. In some embodiments, the use of an appropriate thermosetting polymer and an appropriate copolymer (e.g., a gellling agent) in combination with a specific pH range for the formulation allows for high temperature sterilization of formulations described with substantially no degradation of the therapeutic agent or the polymeric excipients. An advantage of the methods of sterilization provided herein is that, in certain instances, the formulations are subjected to terminal sterilization via autoclaving without any loss of the active agent and/or excipients and/or polymeric components during the sterilization step and are rendered substantially free of microbes and/or pyrogens.

*Microorganisms*

Provided herein are auris-acceptable compositions or devices that ameliorate or lessen otic disorders described herein. Further provided herein are methods comprising the administration of said otic compositions. In some embodiments, the compositions or devices are substantially free of microorganisms. Acceptable bioburden or sterility levels are based on applicable standards that define therapeutically acceptable compositions, including but not limited to United States Pharmacopeia. For example, acceptable sterility (e.g., bioburden) levels include about 10 colony forming units (cfu) per gram of formulation, about 50 cfu per gram of formulation, about 100 cfu per gram of formulation, about 500 cfu per gram of formulation or about 1000 cfu per gram of formulation. In some embodiments, acceptable bioburden levels or sterility for formulations include less than 10 cfu/mL, less that 50 cfu/mL, less than 500 cfu/mL or less than 1000 cfu/mL microbial agents. In addition, acceptable bioburden levels or sterility include the exclusion of specified objectionable microbiological agents. By way of example, specified objectionable microbiological agents include but are not limited to Escherichia coli (E. coli), Salmonella sp., Pseudomonas aeruginosa (P. aeruginosa) and/or other specific microbial agents. In certain embodiments, any controlled release formulation described herein has less than about 60 colony forming units (CFU), less than about 50 colony forming units, less than about 40 colony forming units, or less than about 30 colony forming units of microbial agents per gram of formulation.

Sterility of the auris-acceptable otic therapeutic agent formulation is confirmed through a sterility assurance program in accordance with United States Pharmacopeia. Sterility testing, by way of example only, is performed by two methods. The first is direct inoculation wherein a sample of the composition to be tested is added to growth medium and incubated for a period of time up to 21 days. Turbidity of the growth medium indicates contamination. Drawbacks to this method include the small sampling size of bulk materials which reduces sensitivity, and detection of microorganism growth based on a visual observation. An alternative method is membrane filtration sterility testing. In this method, a volume of product is passed through a small membrane filter paper. The filter paper is then placed into media to promote the growth of microorganisms. This method has the advantage of greater sensitivity as the entire bulk product is sampled. The commercially available Millipore Steritest sterility testing system is optionally used for determinations by membrane filtration sterility testing. For the filtration testing of creams or ointments Steritest filter system No. TLHVSL210 are used. For the filtration testing of emulsions or viscous products Steritest filter system No. TLAREM210 or TDAREM210 are

used. For the filtration testing of pre-filled syringes Steritest filter system No. TTHASY210 are used. For the filtration testing of material dispensed as an aerosol or foam Steritest filter system No. TTHVA210 are used. For the filtration testing of soluble powders in ampoules or vials Steritest filter system No. TTHADA210 or TTHADV210 are used.

Testing for E. coli and Salmonella includes the use of lactose broths incubated at 30 - 35 °C for 24-72 hours, incubation in MacConkey and/or EMB agars for 18-24 hours, and/or the use of Rappaport medium. Testing for the detection of P. aeruginosa includes the use of NAC agar. United States Pharmacopeia Chapter <62> further enumerates testing procedures for specified objectionable microorganisms.

In certain embodiments, the otic formulations described herein are formulated to be suitable for contact with the perilymph in the inner ear (i.e., the formulations are sterile and do not cause infections in the isolated environment of the inner ear).

*Endotoxins*

Provided herein are otic compositions that ameliorate or lessen otic disorders described herein. Further provided herein are methods comprising the administration of said otic compositions. In some embodiments, the compositions or devices are substantially free of endotoxins. An additional aspect of the sterilization process is the removal of byproducts from the killing of microorganisms. The process of depyrogenation removes pyrogens from the sample. Pyrogens are endotoxins or exotoxins which induce an immune response. An example of an endotoxin is the lipopolysaccharide (LPS) molecule found in the cell wall of gram-negative bacteria. While sterilization procedures such as autoclaving or treatment with ethylene oxide kill the bacteria, the LPS residue induces a proinflammatory immune response, such as septic shock. Because the molecular size of endotoxins can vary widely, the presence of endotoxins is expressed in "endotoxin units" (EU). One EU is equivalent to 100 picograms of E. coli LPS. Humans can develop a response to as little as 5 EU/kg of body weight. The bioburden (e.g., microbial limit) and/or sterility (e.g., endotoxin level) is expressed in any units as recognized in the art.

In some embodiments, an auris-acceptable formulation provided herein has endotoxin units (EU) of no more than about 5 EU/kg body weight. By way of example, in some of such embodiments, the total body burden for a typical human weighing 60 kg is no more than about 300 EU. In some embodiments, an auris-acceptable formulation provided herein has endotoxin units (EU) of no more than about 4 EU/kg body weight. By way of example, in some of such embodiments, the total body burden for a typical human weighing 60 kg is no more than about 240 EU. In some embodiments, an auris-acceptable formulation provided herein has endotoxin units (EU) of no more than about 3 EU/kg body weight. By way of example, in some of such embodiments, the total body burden for a typical human weighing 60 kg is no more than about 180 EU. In some embodiments, an auris-acceptable formulation provided herein has endotoxin units (EU) of no more than about 2 EU/kg body weight. By way of example, in some of such embodiments, the total body burden for a typical human weighing 60 kg is no more than about 120 EU. In some embodiments, an auris-acceptable formulation provided herein has endotoxin units (EU) of no more than about 1 EU/kg body weight. By way of example, in some of such embodiments, the total body burden for a typical human weighing 60 kg is no more than about 60 EU. In some embodiments, an auris-acceptable formulation provided herein has endotoxin units (EU) of no more than about 0.5 EU/kg body weight. By way of example, in some of such embodiments, the total body burden for a typical human weighing 60 kg is no more than about 30 EU. In some embodiments, an auris-acceptable formulation provided herein has endotoxin units (EU) of no more than about 0.2 EU/kg body weight. By way of example, in some of such embodiments, the total body burden for a typical human weighing 60 kg is no more than about 12 EU.

In some embodiments, an auris-acceptable formulation provided herein has endotoxin units (EU) of no more than about 60 EU per 0.2 mL of the formulation. By way of example, in some of such embodiments, the total body burden for a 0.2 mL injection is about 60 EU. In some embodiments, an auris-acceptable formulation provided herein has endotoxin units (EU) of no more than about 40 EU per 0.2 mL of the formulation. In some embodiments, an auris-acceptable formulation provided herein has endotoxin units (EU) of no more than about 20 EU per 0.2 mL of the formulation. In some embodiments, an auris-acceptable formulation provided herein has endotoxin units (EU) of no more than about 15 EU per 0.2 mL of the formulation. In some embodiments, an auris-acceptable formulation provided herein has endotoxin units (EU) of no more than about 10 EU per 0.2 mL of the formulation.

Pyrogen detection, by way of example only, is performed by several methods. Suitable tests for sterility include tests described in United States Pharmacopoeia (USP) <71> Sterility Tests (23rd edition, 1995). The rabbit pyrogen test and the Limulus amebocyte lysate test are both specified in the United States Pharmacopeia Chapters <85> and <151> (USP23/NF 18, Biological Tests, The United States Pharmacopeial Convention, Rockville, MD, 1995). Alternative pyrogen assays have been developed based upon the monocyte activation-cytokine assay. Uniform cell lines suitable for quality control applications have been developed and have demonstrated the ability to detect pyrogenicity in samples that have passed the rabbit pyrogen test and the Limulus amebocyte lysate test (Taktak et al, J. Pharm. Pharmacol. (1990), 43:578-82). In an additional embodiment, the auris-acceptable otic formulation is subject to depyrogenation. In a further embodiment, the process for the manufacture of the auris-acceptable otic formulation comprises testing the

formulation for pyrogenicity. In certain embodiments, the formulations described herein are substantially free of pyrogens.

**Tunable Release Characteristics**

**Suspensions**

In some embodiments, a thermoreversible gel formulation comprising a dissolved active agent (e.g., a dissolved corticosteroid) rapidly dumps its drug payload, which is then cleared from auditory structures, thereby reducing the duration of release of the drug. Accordingly, in specific embodiments, any formulation described herein (e.g., a formulation comprising a corticosteroid) comprises a suspension of multiparticulate active agent, i.e., a plurality of undissolved active agent particles (e.g., micronized particles, nano-sized particles, non-sized particles, colloidal particles); i.e, the formulation is a multiparticulate suspension formulation.

As used herein, a "multiparticulate suspension formulation" or "multiparticulate formulation" refers to a formulation comprising at least some undissolved active agent. For example, in one embodiment, a multiparticulate suspension formulation comprises a portion of an active agent in dissolved form and also insoluble or poorly soluble or encapsulated active agent. As used herein, "undissolved" or "encapsulated" or "insoluble" or "poorly soluble" active agent particles are particles having a property of slow dissolution in a medium (e.g., buffered water, buffered poloxamer solution, body fluids, perilymph, middle ear fluid and the like). In other words, insoluble or encapsulated or undissolved or poorly soluble active agent particles are only slightly soluble in a medium (e.g., buffered water, buffered poloxamer solution,body fluids, perilymph, middle ear fluid and the like) and remain at the site of administration for a longer period of time serving as a depot for release of an active agent via slow dissolution over a period of time.

In some of the above embodiments, the undissolved or encapsulated or insoluble or poorly soluble form of the active agent is chosen in order to achieve tunable sustained release as described in more detail below. By way of example, use of a larger particle size, or a crystal form having poor solubility, or a sparingly soluble salt form or a free base allows for the presence of at least some undissolved active agent in a multiparticulate suspension formulation.

In specific embodiments, upon administration of a sustained release suspension formulation comprising insoluble multiparticulate active agent (e.g., multiparticulate dexamethasone, multiparticulate dexamethasone acetate, multiparticulate prednisolone, multiparticulate methylprednisolone or the like) to an individual in need thereof, the active agent particles serve as a depot for further extended release of the active agent even after the gel has eroded. In some of such embodiments, the multiparticulate active agent is a micronized powder. In some of such embodiments, the particles remain adhered to auditory surfaces. Accordingly, in some embodiments, sustained release pharmaceutical formulations suitable for methods described herein comprise substantially high concentrations of multiparticulate insoluble active agent particles. In some of such embodiments, sustained release pharmaceutical formulations described herein (e.g., corticosteroid formulations) are multiparticulate suspensions comprising micronized active agents.

In some embodiments, the use of multiparticulate active agent (e.g., insoluble corticosteroid) allows for extended and/or sustained release of the active agent from any formulation described herein compared to a formulation comprising non-multiparticulate or a water-soluble active agent. In some instances, the multiparticulate and/or less water-soluble active agent provides a steady supply (e.g., +/- 20%) of active agent via slow degradation and/or dissolution and serves as a depot for the active agent; such a depot effect increases residence time of the active agent in the ear. In specific embodiments, selection of an appropriate particle size of the active agent (e.g., corticosteroids) and solubility of the active agent in water (e.g., an insoluble form of dexamethasone, dexamethasone acetate, prednisolone or methylprednisolone), in combination with the amount of thermosensitive polymer component in the composition, provides tunable extended release characteristics that allow for release of an active agent over a period of hours, days, weeks or months.

**Modulation of solubility of an active agent**

The solubility of an active agent (e.g., a corticosteroid such as dexamethasone, dexamethasone acetate, methylprednisolone, prednisolone and the like) from a formulation described herein is modified in biological and/or aqueous media to allow for sustained release from the formulation. One approach to extend release of an active agent is to desolubilize the soluble active agent. Solubility of the drug in biological and/or aqueous fluids is modified by selection of a pharmacologically acceptable salt that is insoluble or has a lower solubility than the drug alone or a different salt of the drug. In certain instances, solubility of the drug in biological and/or aqueous fluids is modified by selection of crystalline salt forms (polymorphs) that are insoluble or have lower solubility than other salt forms or the drug alone.

By way of example, in the case of anionic drugs (e.g., active agents bearing acidic moieties like carboxylic acids, phosphates, sulfates, or the like) a soluble drug is rendered insoluble or less soluble in biological and/or aqueous fluids by exchanging the counterion from a Group I metal ion (e.g., sodium or potassium), to a counterion from group II of the periodic table (e.g., calcium or magnesium) or any other polyvalent cation (e.g., iron, zinc, barium, cesium or the like). By way of example, for cationic drugs (e.g., active agents containing primary, secondary, or tertiary aliphatic or

aromatic amines), a soluble drug is rendered insoluble or less soluble in biological and/or aqueous fluids by formulating at or above the pKa of at least one of the amine moieties. By way of example, for a pKa of ~ 5 for an amine moiety in a drug, a formulation having a pH >5 reduces the solubility of the drug in biological and/or aqueous fluids. By way of example, when an active agent is a cationic drug (e.g., an agent bearing at least one amine moiety with a pKa~5), a poloxamer gel formulation at a pH of 4.5 has a lower mean dissolution time (MDT) compared to a poloxamer formulation at a pH of 7.4.

Further, certain drugs are rendered insoluble or less soluble in biological and/or aqueous media by exchanging the salt of such a drug from a mineral acid salt (e.g., hydrochloric acid or sulfuric acid salts) to a salt of a small to medium sized organic acid (e.g., a citrate, maleate, nicotinate, or besylate salt or the like). By way of example only, a water soluble active agent has a solubility of ≥ 10 mg/ mL. An active agent that has been rendered less soluble or insoluble in aqueous and/or biological media has a water solubility of less than 10 mg/mL, less than 1mg/mL or less than 0.1mg/mL. The release profile of an active agent and/or any salts thereof is compared using suitable in vitro and in vivo procedures.

A second approach for controlling the dissolution and/or release profile of an active agent is to form a complex of an active agent with a complexation agent that hinders dissolution of the active agent in biological and/or aqueous media. Examples of such complexation agents include and are not limited to cryptands (e.g., [2.2.2]cryptand, diaza-18-crown-6), cyclodextrins, crown ethers (e.g., 12-crown-4, 15-crown-5, 18-crown-6, dibenzo-18-crown-6 or the like), or the like. The release profile of an active agent and a complex thereof is compared using in vitro and in vivo procedures described herein.

A further approach to extend the release profile of an active agent from a formulation described herein is to use prodrugs of an active agent. An active agent (anionic, cationic, zwitterionic or neutral) is rendered insoluble or less soluble in biological and/or aqueous media by formation of a prodrug that is insoluble or less soluble in biological and/or aqueous media than the drug alone. Such prodrugs are formed by covalent attachment of a moiety (e.g., an ester, or amide of a bulky or water insoluble group such as benzoic acid, amines, fatty acids, cyclic or aromatic acids or alcohols, polymeric chains, or the like) to the parent drug. The release profile of an active agent and a prodrug thereof is compared using in vitro and in vivo procedures described herein.

**A** further approach to tuning the dissolution properties and/or release profile of an active agent is to coat particles of the active agent with certain sustained release excipients (e.g., hydroxypropylmethyl cellulose, carboxymethylcellulose or the like). By way of example, an active agent is micronized and the micronized particles are coated with sustained release excipients; the coated active agent particulates are then formulated in any of the compositions described herein.

Thus, a combination of an appropriate thermosensitive polymer vehicle and physicochemical properties of a drug (e.g., a multiparticulate corticosteroid, less soluble salt of a corticosteroid) provides an optimized release profile. By way of example, for a 17% Poloxamer 407 formulation, when either dexamethasone or methylprednisolone is present as a water soluble salt, i.e. DSP and MPS, respectively, MDT values are about 3h. However, the MDT values of water insoluble forms of dexamethasone and methylprednisolone (e.g., DEX, DA and MP) range from 40 to 71 h. By way of example, a DSP aqueous solution has a MDT of 0.3h whereas a micronized DEX suspension in water has a MDT value of 44h.

In some embodiments, the solubility of the drug modulates the pharmacokinetics of the formulation. By way of example, intratympanic administration of DSP in hydrogel vehicle in guinea pigs resulted in limited inner ear exposure (AUC values ranging from 28 to 57 μg.h/ml) and rapid elimination from inner ear compartment (MRT of 4-7 h). However, administration of a less soluble form of the drug, i.e., DEX or Dexamethasone acetate (DA) in hydrogel vehicle led to higher dexamethasone exposure in the perilymph (AUC of 84-359 μg.h/ml) and prolonged residence time (MRT 17-82 h).

By way of example, the inner ear profile of methylprednisolone is tunable via the use of soluble (MPS) and water insoluble (MP) forms. Methylprednisolone levels in the perilymph peaked rapidly following intratympanic administration of the MPS hydrogel in guinea pigs at 6.5 μg/ml and decreased to a fraction of the peak levels (0.8-1.0 %) within 3 days. In contrast, administration of a formulation comprising the less soluble MP resulted in higher peak levels (19.2 μg/ml) that decreased slowly over 10 days.

Thus insoluble particles of a corticosteroid (e.g., multiparticulate dexamethasone, dexamethasone acetate, prednisolone, methylprednisolone and the like) in a suspension thermoreversible gel formulation comprising a copolymer of polyoxyethylene and polyoxypropylene increase residence times of the active agent in otic regions (e.g., perilymph).

Yet another approach for tuning the release profile of an active agent is by changing the concentration of an active agent in the formulation. By way of example, at increased concentration of an active agent, a) initial drug levels reached in the inner ear (as measured in perilymph) are high and b) there is an increase in the duration of exposure.

Particle size modulation is optionally used to increase surface area and/or modulate active agent dissolution properties and/or to maintain a consistent average particle size distribution (PSD) (e.g., micrometer-sized particles, nanometer-sized particles or the like) for any formulation described herein. Micronization is a process of reducing the average diameter of particles of a solid material. In some embodiments, the average diameter of particles in a micronized solid is from about 0.5 μm to about 500 μm. In some embodiments, the average diameter of particles in a micronized solid is from about 1 μm to about 200 μm. In some embodiments, the average diameter of particles in a micronized solid

is from about 2 μm to about 100 μm. In some embodiments, the average diameter of particles in a micronized solid is from about 3□m to about 50 □m.

In some instances, any particle in a formulation described herein is a coated or uncoated particle (e.g., a coated micronized particle, nano-particle) and/or a microsphere and/or a liposomal particle. Particle size reduction techniques include, by way of example, grinding, milling (e.g., air-attrition milling (jet milling), ball milling), coacervation, complex coacervation, high pressure homogenization, spray drying and/or supercritical fluid crystallization. In some instances, particles are sized by mechanical impact (e.g., by hammer mills, ball mill and/or pin mills). In some instances, particles are sized via fluid energy (e.g., by spiral jet mills, loop jet mills, and/or fluidized bed jet mills).

The release profile of compositions suitable for methods described herein is optionally determined using dissolution techniques. In one embodiment, dissolution is performed at 37°C in snapwells (6.5 mm diameter polycarbonate membrane with a pore size of 0.4 μm), 0.2 mL of a gel formulation described herein (e.g., a gel formulation of Example 1) is placed into snapwell and left to harden, then 0.5 mL buffer is placed into reservoir and shaken using a Labline orbit shaker at 70 rpm. Samples are taken every hour (0.1 mL withdrawn and replace with warm buffer). Samples are analyzed for active agent concentration by UV at 245nm against an external calibration standard curve. Pluronic concentration is analyzed at 624 nm using the cobalt thiocyanate method. Relative rank-order of mean dissolution time (MDT) as a function of %P407 is determined. A linear relationship between the formulations mean dissolution time (MDT) and the P407 concentration indicates that the active agent is released due to the erosion of the polymer gel (poloxamer) and not via diffusion. A non-linear relationship indicates release of active agent via a combination of diffusion and/or polymer gel degradation.

The MDT is inversely proportional to the release rate of an active agent from a composition described herein. Experimentally, the released active agent is optionally fitted to the Korsmeyer-Peppas equation:

$$\frac{Q}{Q_\alpha} = kt^n + b$$

where Q is the amount of active agent released at time t, Qα is the overall released amount of active agent, k is a release constant of the nth order, n is a dimensionless number related to the dissolution mechanism and b is the axis intercept, characterizing the initial burst release mechanism wherein n=1 characterizes an erosion controlled mechanism. The mean dissolution time (MDT) is the sum of different periods of time the drug molecules stay in the matrix before release, divided by the total number of molecules and is optionally calculated by:

$$MDT = \frac{nk^{-1/n}}{n+1}$$

In alternate embodiments, samples are analyzed using the method described by Li Xin-Yu paper [Acta Pharmaceutica Sinica 2008,43(2):208-203] and Rank-order of mean dissolution time (MDT) as a function of %P407 is determined.

Any combination of the active agents and compositions and methods described above for the various variables is contemplated herein. Throughout the specification, agents and compositions and methods for the use thereof are provided, and are chosen by one skilled in the field to provide suitable treatment for individuals in need thereof.

## EXAMPLES

Example 1 - Preparation of a Thermoreversible Gel 2% Dexamethasone Composition comprising multiparticulate dexamethasone

Table A

| Ingredient | Quantity (mg/g of formulation) |
|---|---|
| dexamethasone | 20.0 |
| Poloxamer 407 | 160.0 |
| PBS buffer (0.1 M) | 9.0 |

10-g batch of gel formulation containing 2.0% micronized dexamethasone is prepared. 13.8 mg of sodium phosphate dibasic dihydrate USP (Fisher Scientific.) + 3.1 mg of sodium phosphate monobasic monohydrate USP (Fisher Scientific.) + 74 mg of sodium chloride USP (Fisher Scientific.) is dissolved with 8.2g of sterile filtered DI water and the pH is adjusted to 7.4 with 1 M NaOH. The buffer solution is chilled down and a suitable amount of poloxamer 407 (BASF Corp., containing approximately 100 ppm of BHT) is sprinkled into the chilled PBS solution while mixing, the solution is mixed until all the poloxamer is dissolved. The poloxamer is sterile filtered using a 33mm PVDF 0.22μm sterile syringe filter (Millipore Corp.) and delivered to 2 mL sterile glass vials (Wheaton) in an aseptic environment, the vials are closed with sterile butyl rubber stoppers (Kimble) and crimped sealed with 13 mm Al seals (Kimble). 20 mg of micronized dexamethsone is placed in separate clean depyrogenated vials, the vials are closed with sterile butyl rubber stoppers (Kimble) and crimped sealed with 13 mm Al seals (Kimble), vials are dry heat sterilized (Fisher Scientific Isotemp oven) for 7 hours at 140°C. Before administration for the experiments described herein, 1 mL of the cold poloxamer solution is delivered to a vial containing 20 mg of sterile micronized dexamethasone using a 21G needle (Becton Dickinson) attached to a 1 mL sterile syringe (Becton Dickinson), suspension mixed well by shaking to ensure homogeneity of the suspension. The suspension is then withdrawn with the 21G syinge and the needle is switched to a 27 G needle for administration.

A 6% dexamethasone formulation was also prepared as a ready to use product as follows: Weigh 205.4 g of DI water, then add 1.1342 g of sodium chloride (Fisher scientific), add 1.53g of tromethamine (Fisher scientific), dissolve and adjust pH to 7.8 with approximately 1.9mL of 5 N HCl. Weigh 126.2g of buffer and chill down, sprinkle 24.5g of poloxamer 407 (Lutrol F127, BASF) while mixing to dissolve. Take 6.2 mL of 16% poloxamer 407 solution and add 400mg of micronized dexamethasone (Pfizer) to the mixture while mixing, transfer one mL to 2mL autoclaved vials and autoclave them at 121°C for 30 minutes.

Examples 2-5 Preparation of Thermoreversible Gel Compositions comprising multiparticulate corticosteroids

Thermoreversible gel formulations comprising poloxamer and insoluble particles of erythromycin, prednisolone, methylprednisolone and triamcinolone respectively are prepared using the procedure described in Example 1 above.

Example 6: - Preparation of a Thermoreversible Gel JNK inhibitor Composition

A 2% SP600125 in 16% poloxamer formulation was manufactured as a ready to use product as follows: Weigh 205.4 g of DI water, then add 1.1342 g of sodium chloride (Fisher scientific), add 1.53g of tromethamine (Fisher scientific), dissolve and adjust pH to 7.8 with approximately 1.9mL of 5 N HCl. Weigh 126.2g of buffer and chill down, sprinkle 24.5g of poloxamer 407 (Lutrol F127, BASF) while mixing to dissolve. Sterile filter the 16% poloxamer solution with a 0.22 μm PVDF 33 mm syringe filter. Weigh 207 mg of milled SP600125 (LC laboratories) then add 1.8mL of sterile filtered 16% poloxamer 407, then transfer to 3 mL autoclaved vials.

Example 7 Preparation of a Thermoreversible Gel Dexamethasone Composition comprising micronized dexamethasone powder and purified poloxamer

*Purification of Poloxamer*

Method A: Poloxamer 407 (BASF Corporation, lot WPEB612B) is dissolved in of 75/25 water/iso-propanol v/v solution. The solution is equilibrated to 27 °C. Sodium chloride is added with vigorous mixing and the solution is centrifuged to allow two clear, colorless phases to form. The lower phase is drained and the solution is again diluted to near its initial weight/volume by the addition of water/iso -propanol 75/25 v/v solution followed by equilibration to 27 °C and addition of sodium chloride. The solution is centrifuged to allow two clear, colorless phases to form. The lower phase is drained a second time and the solution returned to near its original weight by the addition of water/iso-propanol solution and sodium chloride as described earlier. The resulting solution is centrifuged, the lower phase is drained and discarded. The upper phase from the third extraction is dried then extracted with chloroform. The chloroform layer is then evaporated in vacuo. The residue is dried under vacuum.

Method B: Poloxamer 407 from BASF Corporation, Mount Olive, N.J., is dissolved in deionized water. The solution is maintained close to freezing, then ammonium sulfate is added. The solution is equilibrated at 2° C. and after two distinct phases are formed, the lower phase is discarded, and the upper phase is collected and weighed. Deionized water is added and the solution is equilibrated to 2°C. followed by addition of ammonium sulfate with stirring. After the salt is dissolved, the solution is maintained at approximately 2° C. until two phases formed. The upper phase is isolated and diluted with deionized water. The solution is chilled to about 2° C. and ammonium sulfate is added.

The phases are allowed to separate as above. The upper phase is isolated and extracted with dichloromethane. Two phases are allowed to form overnight. The organic (lower) phase is isolated and dried over sodium sulfate. The dichloromethane phase is filtered through a PTFE filter (0.45 μm pore size) to remove the undissolved salts. The dichloromethane is removed in vacuo and the residue is dried overnight in an oven.

Following the procedure in Example 1, the purified poloxamer from Method A or Method B above is used for preparation of a formulation comprising the components described in Table B below.

Table B

| Ingredient | Quantity (mg/g of formulation) |
|---|---|
| dexamethasone | 20.0 |
| Purified Poloxamer 407 | 120.0 |
| PBS buffer (0.1 M) | 9.0 |

Example 8 - Preparation of a Thermoreversible Gel IGF-1 Composition

A 0.05% IGF-1 in 17% poloxamer formulation was manufactured as a ready to use product as follows: Weigh 205.4 g of DI water, then add 1.1342 g of sodium chloride (Fisher scientific), add 1.53g of tromethamine (Fisher scientific), dissolve and adjust pH to 7.8 with approximately 1.9mL of 5 N HCl. Weigh 83g of buffer and chill down, sprinkle 17.1g of poloxamer 407 (Lutrol F127, BASF) while mixing to dissolve, then dissolve 5mg of evans blue (Sigma) . Sterile filter the 17% poloxamer solution with a 0.22μm PVDF 33 mm syringe filter. Add 2 mL of the sterile filtered 17% poloxamer solution/evans blue to 1mg of IGF-1 (PeproTech) and dissolve the drug by gentle mixing. Transfer formulation to a 2mL autoclaved vial.

Example 9 - In vivo testing of Intratympanic Injection of dexamethasone formulation in a guinea pig in a cisplatin induced ototoxicity model.

Female guinea pigs (Charles River) weighing 200-300g, of approximately 6-8 weeks of age are used (N = 4 per group). Prior to any procedure, animals are anesthetized using a combination of xylazine (10 mg/kg), ketamine (40 mg/kg) and acepromazine (0.75 mg/kg) for up to an hour via the intramuscular route. If needed, an intraoperative booster is administered intraperitoneally representing one-tenth of the original dose. *Intratympanic injection* - Each animal is positioned so that the head is tilted at an angle to favor injection towards the round window niche. Briefly, under visualization with an operating microscope, 50 μl of formulations comprising 0.2 - 6% dexamethasone and varying concentrations of P407 are admininstered to the animals. The formulations are injected using a 27G or 30G needle through the tympanic membrane into the superior posterior quadrant behind which the round window niche is located. During the procedure and until recovery, animals are placed on a temperature controlled (40 °C) heating pad until consciousness is regained at which time they are returned to the vivarium.

*Perilymph sampling procedure* - The skin behind the ear of anesthetized guinea pigs is shaved and disinfected with povidone-iodine. An incision is then made behind the ear, and muscles are carefully retracted from over the bulla. A hole is drilled though the bulla using a dental burr so that the middle ear is exposed and accessible. The cochlea and the round window membrane are visualized under a stereo surgical microscope. A unique microhole is hand drilled through the bony shell of the cochlea (active capsule) adjacent to the round window. Perilymph (5 μl) is then collected using a microcapillary inserted into the cochlear scala tympani. *Plasma and CSF collection methods* - Blood is collected by cardiac puncture into heparin coated tubes. To collect the cerebrospinal fluid (CSF), a small skin incision is made just posterior to the cranial vertex. The skin is then retracted, and the trapezius muscle scraped off the occipital bone. A small hole is then drilled through the bone. The dura is cut with a sharp scalpel and a micropipette inserted to collect blood-free CSF (50 μl).

*Cisplatin delivery:* Intratympanic injection of a formulation of Example 1 is administered the day before cisplatin administration. The lower right quadrant of the abdomen is shaved 1 inch and swabbed with alcohol. A tiny (2-3 mm) incision is made on the abdomen and a 21 G blunt needle is used to penetrate the abdominal wall and into the intraperitoneal cavity. The needle will be connected to an infusion bag containing cisplatin for a slow infusion of 15-30 minutes in the range of 5-15 mg/kg. The incision site is closed using sterile staples. Animals will be placed on a warming pad during the infusion and while recovering. In addition, animals receive twice daily IP injection of saline solution for 3 days to prevent nephrotoxicity.

Hearing test - The hearing of the animal is tested by recording the brainstem activity in response to a known

auditory stimulus. (ABR: Auditory Brainstem Response) at various time points. This measurement is performed under general anesthesia. During the procedure the animal is placed on a heating pad (40oC) in a sound proof booth and an earphone is fitted loosely into one ear at a time. Three subcutaneous needle electrodes are used to measure the brainstem activity. One is placed behind the ear with the earphone, one on the vertex of the skull and one in the hindleg. The recording then takes place, where the audio stimulus is applied at different frequencies and hearing thresholds, and the brainstem activity recorded.

Examples 10-11: In vivo testing of Intratympanic Injection of methylprednisolone formulation in a guinea pig in an aminoglycoside induced ototoxicity model.

Following the procedure described in Example 9, a methylprednisolone formulation is administered to guinea pigs prior to treatment with Vacomycin or gentamicin. Hearing is evaluated as described in Example 9 above.

Example 12: In vivo testing of Intratympanic Injection of prednisolone formulation in a guinea pig in vincristine induced ototoxicity model.

Following the procedure described in Example 9, a prednisolone formulation is administered to guinea pigs prior to treatment with vincristine. Hearing is evaluated as described in Example 9 above.

Example 13: In vivo testing of Intratympanic Injection of dexamethasone formulation in a guinea pig in acoustic trauma model.

Following the procedure described in Example 9, a dexamethasone formulation is administered to guinea pigs prior to exposure to acoustic trauma. Hearing is evaluated as described in Example 8 above. Figures 2-4 illustrate the protective effect of prophylactic administration of a dexamethasone thermoreversible gel formulation in preventing hearing loss.

Example 14: In vivo testing of Intratympanic Injection of SP600125 formulation in a guinea pig in acoustic trauma model.

Following the procedure described in Example 9, a SP600125 formulation is administered to guinea pigs prior to exposure to acoustic trauma. Hearing is evaluated as described in Example 9 above. Figure 5 shows the effect of JNK inhibitor SP600125 in preventing hearing loss due to acoustic trauma.

Example 15: Clinical trial to test protective effect of intratympanic thermoreversible gel formulation comprising dexamethasone in patients undergoing cisplatin treatment

*Study Aim:* The aim of this study is to examine whether ototoxicity due to cisplatin treatment can be prevented by use of a composition as described in Example 1. Patients with a diagnosis of cancer and prescribed treatment with cisplatin will be enrolled in the study.

*Study Type:* Interventional

*Study Design:* Randomized efficacy study, placebo control. Patients are randomized to 1 of 2 treatment arms, a placebo arm and a dexamethasone treatment arm. A single intratympanic injection of a thermoreversible gel formulation comprising dexamethasone will be administered 24 hours prior to start of cisplatin treatment.

*Primary Outcome Measures:* Threshold hearing levels. Ototoxicity is defined as an increase in the auditory threshold by at least 20 dB at any one test frequency, or at least 10 dB at any two adjacent frequencies, or loss of response at three consecutive frequencies between the baseline and during follow-up studies.

Examples 16-17: Clinical trial to test protective effect of intratympanic thermoreversible gel formulation comprising prednisolone or methylprednisolone

Formulations comprising methylprednisolone or prednisolone are tested in patients undergoing treatment with carboplatin and vancomycin respectively according to the protocol described in Example 15 above. Threshold hearing levels are recorded.

Example 18: Clinical trial to test protective effect of intratympanic thermoreversible gel formulation comprising dexamethasone prior to exposure to acoustic trauma

*Study Aim:* The aim of this study is to examine whether hearing loss due to acoustic trauma can be prevented by use of a composition as described in Example 1. Subjects who anticipate being exposed to loud noise will be enrolled in this study.

*Study Type:* Interventional

*Study Design:* Randomized efficacy study, placebo control. Patients are randomized to 1 of 2 treatment arms, a placebo arm and a dexamethasone treatment arm. A single intratympanic injection of a thermoreversible gel formulation comprising dexamethasone will be administered 24 hours prior to onset of acoustic trauma.

*Primary Outcome Measures:* Threshold hearing levels. Hearing loss is defined as an increase in the auditory threshold by at least 20 dB at any one test frequency, or at least 10 dB at any two adjacent frequencies, or loss of response at three consecutive frequencies between the baseline and during follow-up studies.

Example 19: Clinical trial to test protective effect of intratympanic thermoreversible gel formulation comprising JNK inhibitor prior to exposure to acoustic trauma

A formulation comprising SP600125 is tested in individual who anticipates exposure to acoustic trauma according to the protocol described in Example 18 above. Threshold hearing levels are recorded.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A pharmaceutical composition comprising a multiparticulate corticosteroid or a multiparticulate JNK inhibitor, for use in a method of prevention of drug-induced ototoxicity, wherein the composition is a gel or a viscous preparation, and wherein the pharmaceutical composition is administered to an individual in need thereof prior to onset of treatment with an ototoxicity-inducing drug.

2. The pharmaceutical composition for the use of claim 1, wherein the drug-induced ototoxicity is hearing loss.

3. The pharmaceutical composition for the use of claim 1 or 2, wherein the drug-induced ototoxicity is chemotherapy-induced ototoxicity, and wherein the chemotherapeutic agent that induces ototoxicity is a platinum based chemotherapeutic agent, a bis-platinate, vincristine, an aminoglycoside antibiotic, a macrolide antibiotic, a diuretic or a salicylate.

4. The pharmaceutical composition for the use of claim 3, wherein the platinum based chemotherapeutic agent is cisplatin, carboplatin or oxiplatin.

5. The pharmaceutical composition for the use of claim 3, wherein the bis-platinate is CT-47613 or CT-47609.

6. The pharmaceutical composition for the use of claim 3, wherein the chemotherapeutic agent that induces ototoxicity is vincristine.

7. The pharmaceutical composition for the use of claim 3, wherein the aminoglycoside antibiotic is gentamicin, streptomycin, kanamycin, amikacin or neomycin.

8. The pharmaceutical composition for the use of claim 3, wherein the macrolide antibiotic is erythromycin, azithromycin or clindamycin.

9. The pharmaceutical composition for the use of claim 1, wherein the composition has a gelation temperature of

between about 15 °C and about 42 °C.

**10.** The pharmaceutical composition for the use of claim 1, wherein the multiparticulate JNK inhibitor is selected from minocycline; SB-203580 (4-(4-Fluorophenyl)-2-(4-methylsulfinyl phenyl)-5-(4-pyridyl) 1H-imidazole); PD 169316 (4-(4-Fluorophenyl)-2-(4-nitrophenyl)-5-(4-pyridyl)-1H-imidazole); SB 202190 (4-(4-Fluorophenyl)-2-(4-hydroxyphenyl)-5-(4-pyridyl)1H-imidazole); RWJ 67657 (4-[4-(4-fluorophenyl)-1-(3-phenylpropyl)-5-(4-pyridinyl)-1H-imidazol-2-yl]-3-butyn-1-ol); SB 220025 (5-(2-Amino-4-pyrimidinyl)-4-(4-fluorophenyl)-1-(4-piperidinlyl)imidazole); AM-111; and SP600125.

**11.** The pharmaceutical composition for the use of claim 1, wherein the multiparticulate JNK inhibitor is SP600125.

**12.** The pharmaceutical composition for the use of claim 1, wherein the pharmaceutical composition comprises a polyoxyethylene-polyoxypropylene triblock copolymer.

**13.** The pharmaceutical composition for the use of claim 12, wherein the polyoxyethylene-polyoxypropylene triblock copolymer is Poloxamer 407.

**14.** The pharmaceutical composition for the use of claim 1, wherein the multiparticulate JNK inhibitor is micronized JNK inhibitor.

**15.** The pharmaceutical composition for the use of claim 1, wherein the composition is administered to an individual in need thereof 24 hours prior to onset of treatment with an ototoxicity-inducing drug.

**16.** A method for preventing drug-induced ototoxicity in an individual in need thereof comprising intratympanic administration of a pharmaceutical composition comprising a multiparticulate corticosteroid to the individual in need thereof, wherein the pharmaceutical composition is administered prior to onset of therapy with the drug, and wherein the composition provides sustained release of the corticosteroid into the ear for a period of at least 5 days after a single administration.

**17.** The method of claim 16, wherein the drug-induced ototoxicity is hearing loss.

**18.** The method of claim 17, wherein the drug-induced ototoxicity is chemotherapy- induced ototoxicity.

**19.** The method of claim 18, wherein the chemotherapeutic agent that induces ototoxicity is a platinum based chemotherapeutic agent, a bis-platinate, vincristine, an aminoglycoside antibiotic, a macrolide antibiotic, a diuretic or a salicylate.

**20.** The method of claim 19, wherein the platinum based chemotherapeutic agent is cis-platin, carboplatin or oxiplatin.

**21.** The method of claim 19, wherein the bis-platinate is CT-47613 or CT-47609.

**22.** The method of claim 19, wherein the chemotherapeutic agent that induces ototoxicity is vincristine.

**23.** The method of claim 19, wherein the aminoglycoside antibiotic is gentamicin, streptomycin, kanamycin, amikacin or neomycin.

**24.** The method of claim 19, wherein the macrolide antibiotic is erythromycin, azithromycin or clindamycin.

**25.** The method of claim 16, wherein the composition comprises a gel or a viscous preparation.

**26.** The method of claim 16, wherein the composition comprises a thermoreversible gel.

**27.** The method of claim 26, wherein the thermoreversible gel comprises a copolymer of polyoxy ethylene and polyoxypropylene in an amount sufficient to provide a gelation temperature of between about 15 °C and about 42 °C.

**28.** The method of claim 16, wherein the corticosteroid is selected from dexamethasone, dexamethasone acetate, prednisone and methylprednisolone, or pharmaceutically acceptable salt thereof.

**29.** A method for preventing hearing loss due to acoustic trauma in an individual in need thereof comprising intratympanic administration of a pharmaceutical composition comprising a multiparticulate JNK inhibitor to the individual in need thereof, wherein the pharmaceutical composition is administered prior to onset of acoustic trauma, and wherein the composition provides sustained release of the JNK inhibitor into the ear for a period of at least 5 days after a single administration.

**30.** The method of claim 28, wherein the JNK inhibitor is selected from minocycline; SB-203580 (4-(4-Fluorophenyl)-2-(4-methylsulfinyl phenyl)-5-(4-pyridyl) 1H-imidazole); PD 169316 (4-(4-Fluorophenyl)-2-(4-nitrophenyl)-5-(4-pyridyl)-1H-imidazole); SB 202190 (4-(4-Fluorophenyl)-2-(4-hydroxyphenyl)-5-(4-pyridyl)1H-imidazole); RWJ 67657 (4-[4-(4-fluorophenyl)-1-(3-phenylpropyl)-5-(4-pyridinyl)-1H-imidazol-2-yl]-3-butyn-1-ol); SB 220025 (5-(2-Amino-4-pyrimidinyl)-4-(4-fluorophenyl)-1-(4-piperidinlyl)imidazole); AM-111; and SP600125.

**31.** The method of claim 28, wherein the JNK inhibitor is SP600125.

**32.** The method of claim 28, wherein the composition comprises a gel or a viscous preparation.

**33.** The method of claim 28, wherein the composition comprises a thermoreversible gel.

**34.** The method of claim 33, wherein the thermoreversible gel comprises a copolymer of polyoxy ethylene and polyoxypropylene polyoxypropylene in an amount sufficient to provide a gelation temperature of between about 15 °C and about 42 °C.

FIG. 1

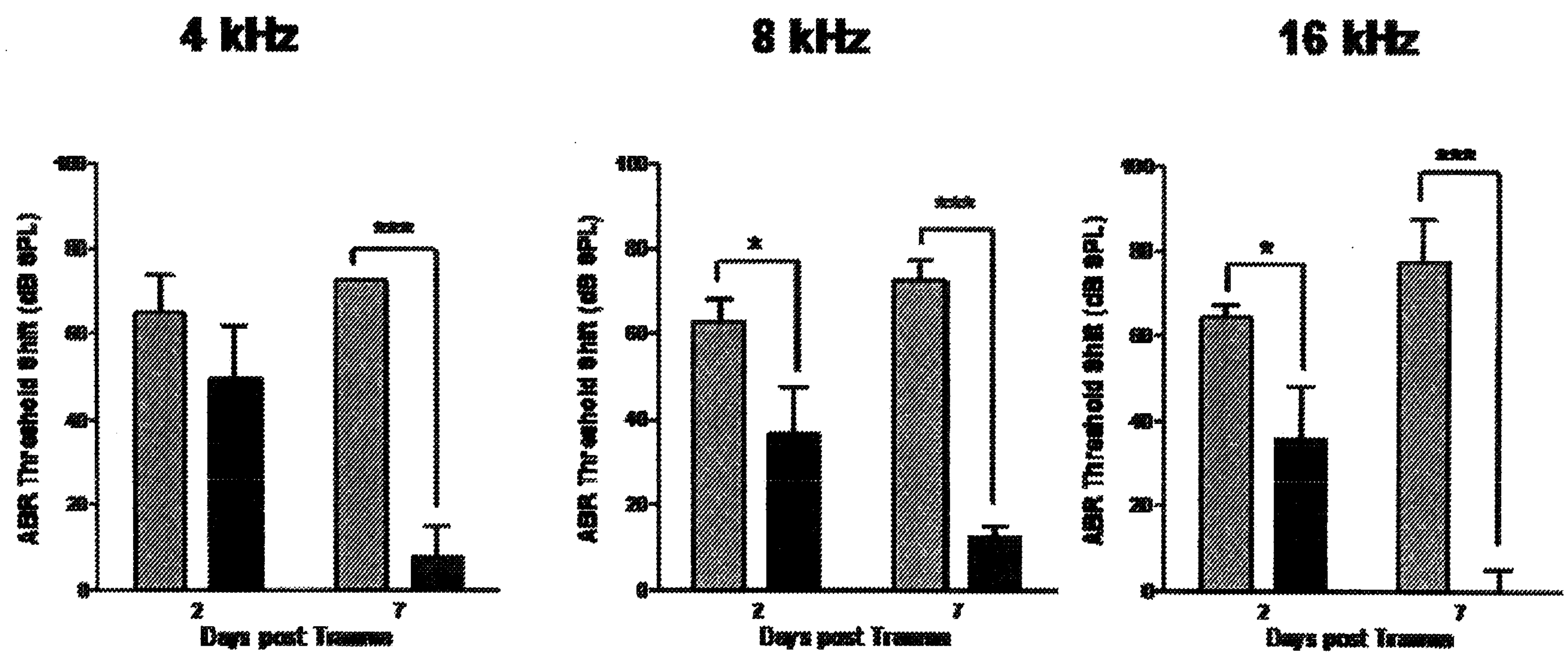


Intratympanic injection (50 µl) given 24h prior to cisplatin treatment (12 mg/kg) (n = 6)
ABR at low (4 kHz), mid (8 kHz) and high (16 kHz) frequencies
Gray bars : Poloxamer vehicle Black bars: 6% Dex

FIG. 2

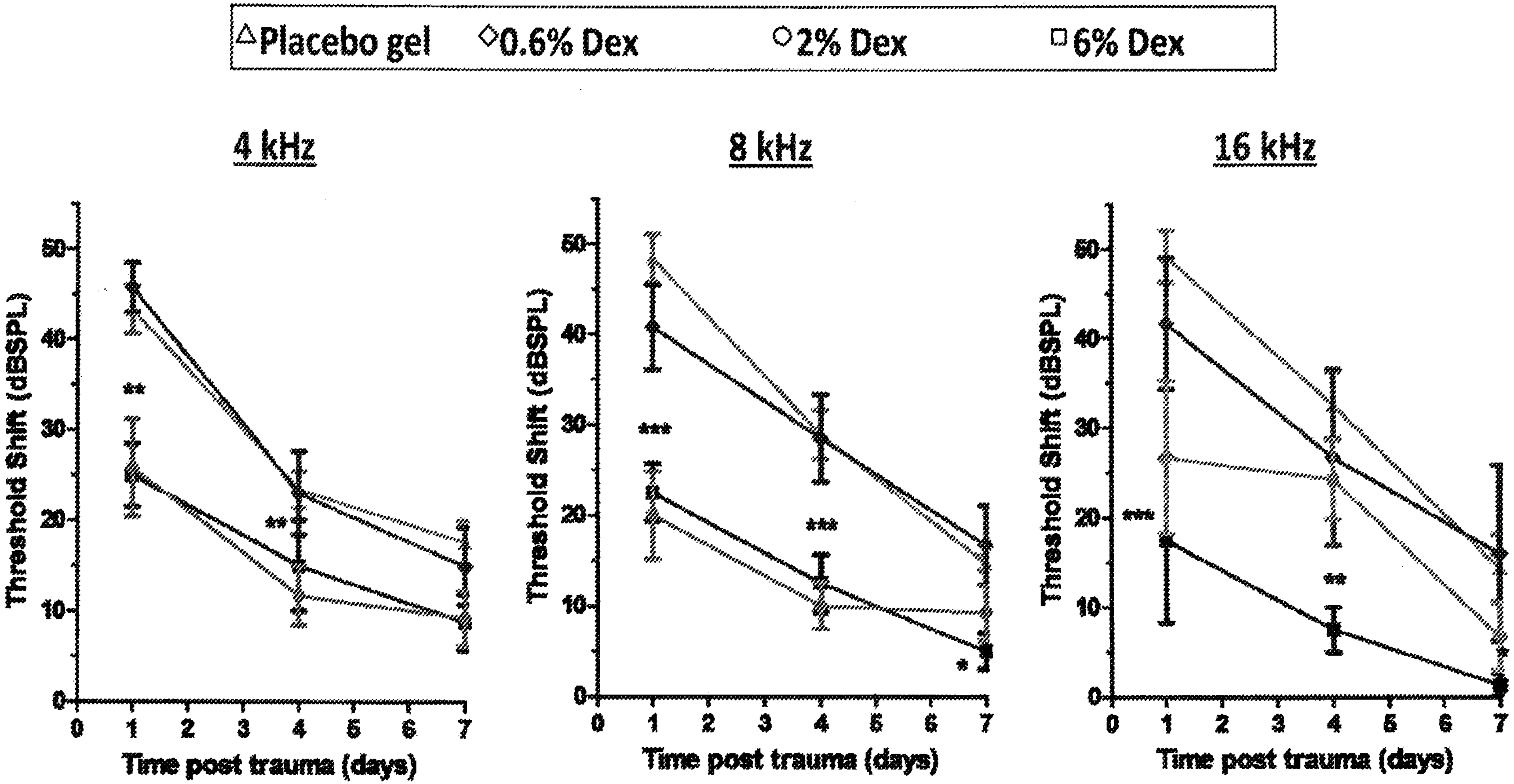


Intratympanic injection (50 µl) given 24 h prior to noise exposure (n = 6)
ABR = auditory brainstem response (higher level = worse hearing loss)
Trauma : 105 dB with a bandpass of 4-8KHz, 2h
P values: * <0.05, ** <0.01, *** <0.001

FIG. 3

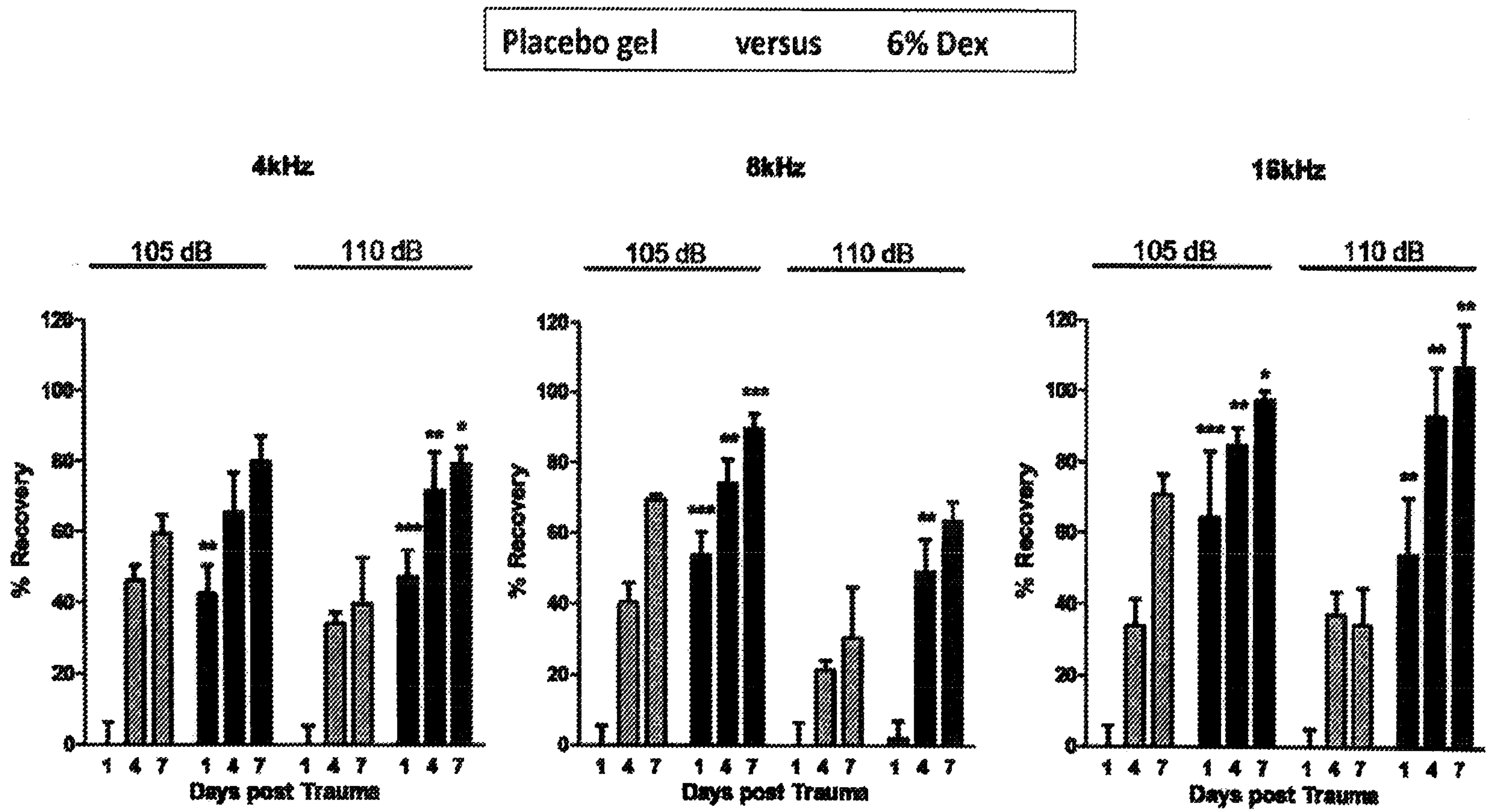


Intratympanic injection (50 µl) given 24 h prior to noise exposure (n = 6)
Placebo gel (gray column) and 6% Dex (black column)
ABR = auditory brainstem response (higher level = worse hearing loss)
Trauma : 105 or 110 dB with a narrow bandpass of 4-8KHz, 2h
P values: * <0.05, ** <0.01, *** <0.001

FIG. 4

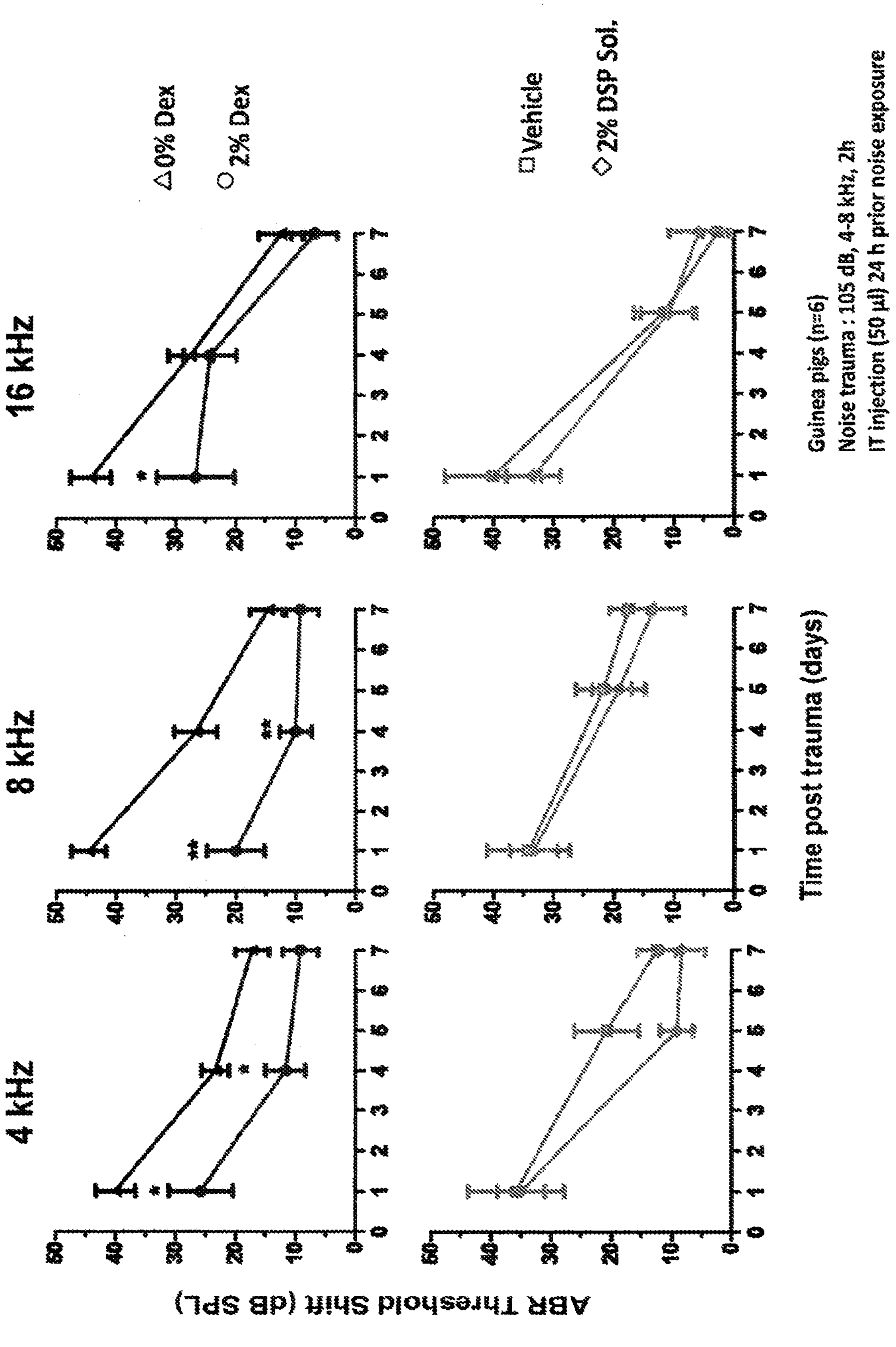
4 kHz
8 kHz
16 kHz
△0% Dex
○2% Dex
□Vehicle
◇2% DSP Sol.
ABR Threshold Shift (dB SPL)
Time post trauma (days)
Guinea pigs (n=6)
Noise trauma : 105 dB, 4-8 kHz, 2h
IT injection (50 µl) 24 h prior noise exposure

FIG. 5

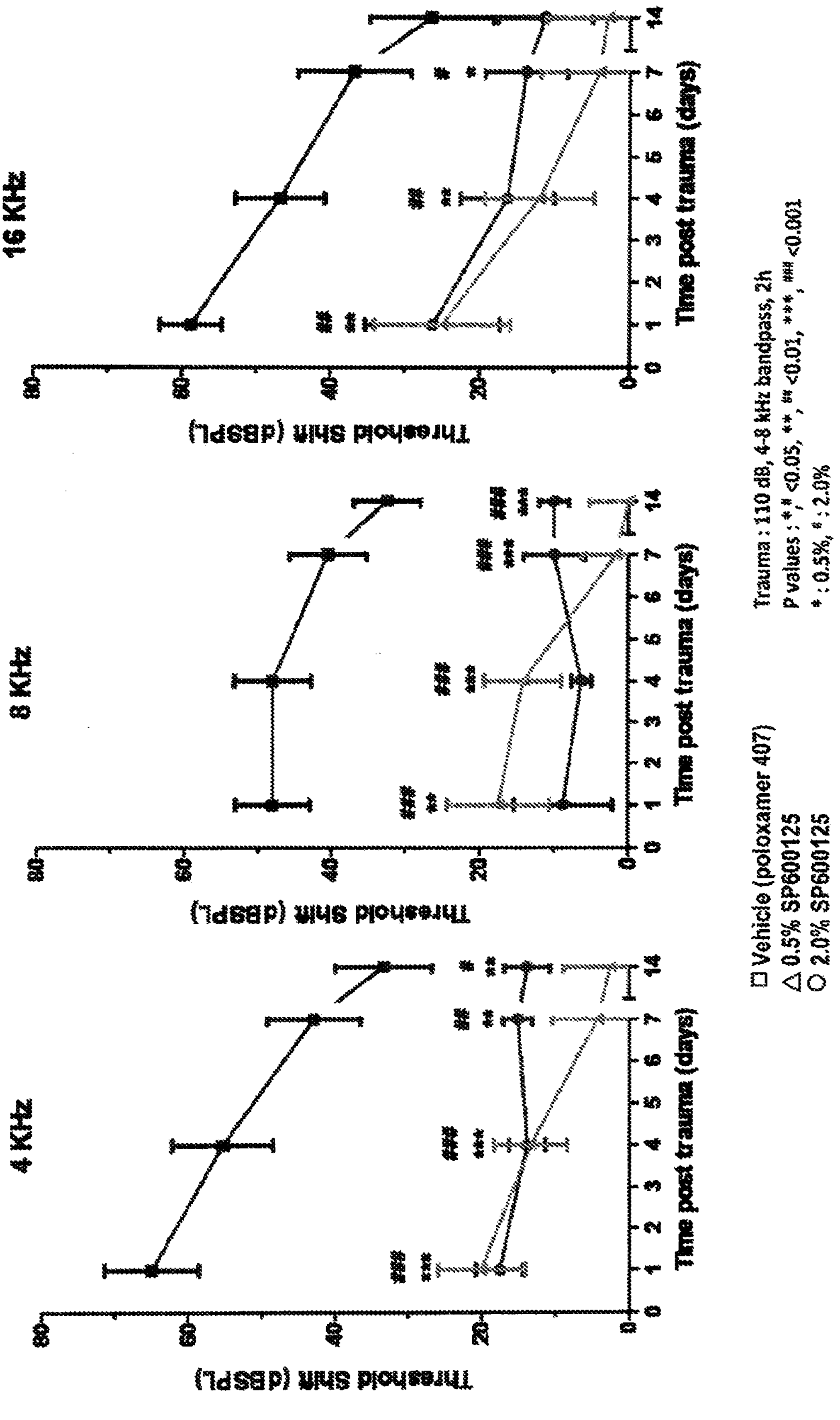
4 KHz
8 KHz
16 KHz
Threshold Shift (dBSPL)
Time post trauma (days)
□ Vehicle (poloxamer 407)
△ 0.5% SP600125
○ 2.0% SP600125
Trauma : 110 dB, 4-8 kHz bandpass, 2h
P values : *,# <0.05, **,## <0.01, ***,### <0.001
* : 0.5%, # : 2.0%

FIG. 6

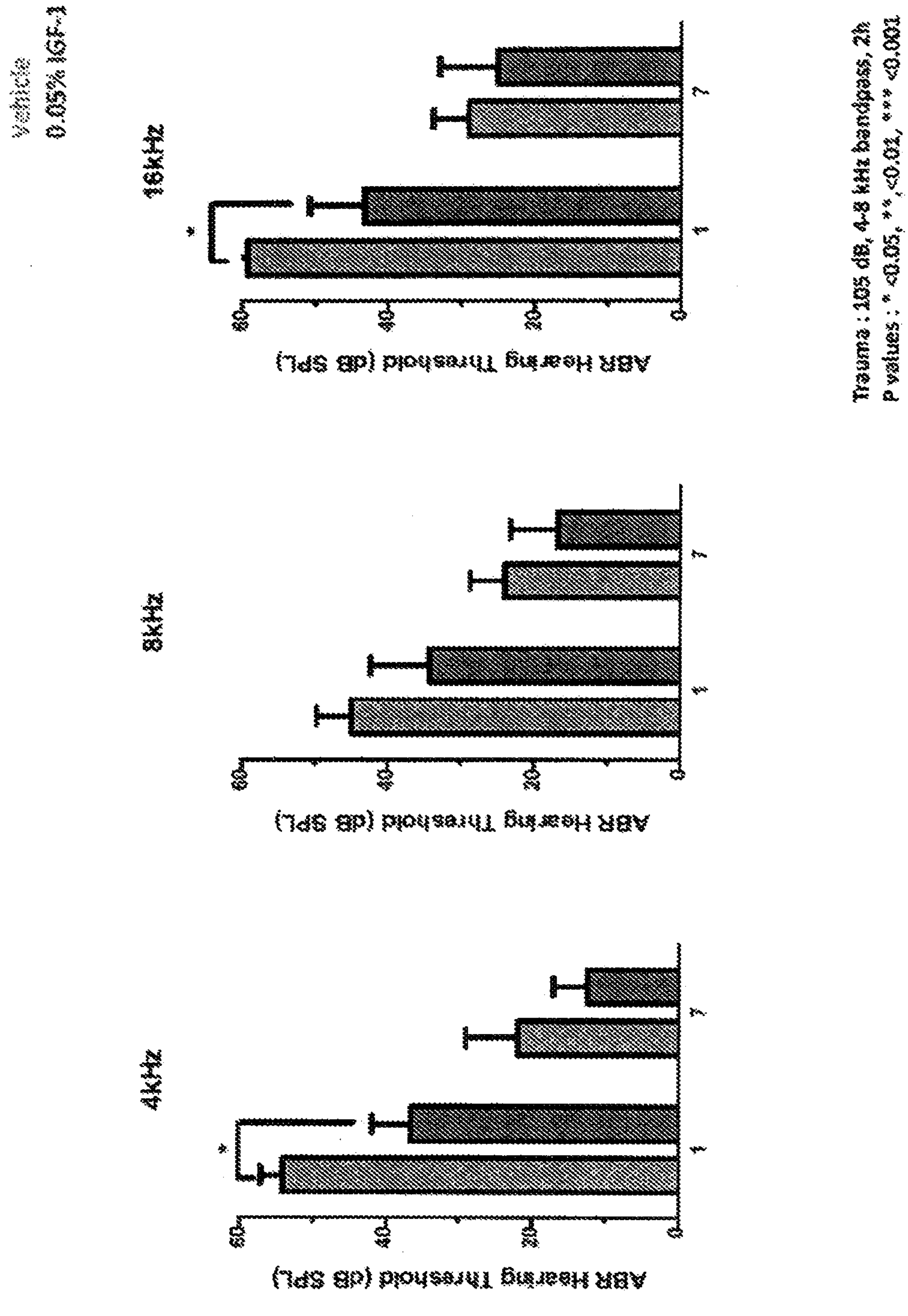
Vehicle
0.05% IGF-1
4kHz
8kHz
16kHz
ABR Hearing Threshold (dB SPL)
0
20
40
60
1
7
*
Trauma : 105 dB, 4-8 kHz bandpass, 2h
P values : * <0.05, ** <0.01, *** <0.001

FIG. 7

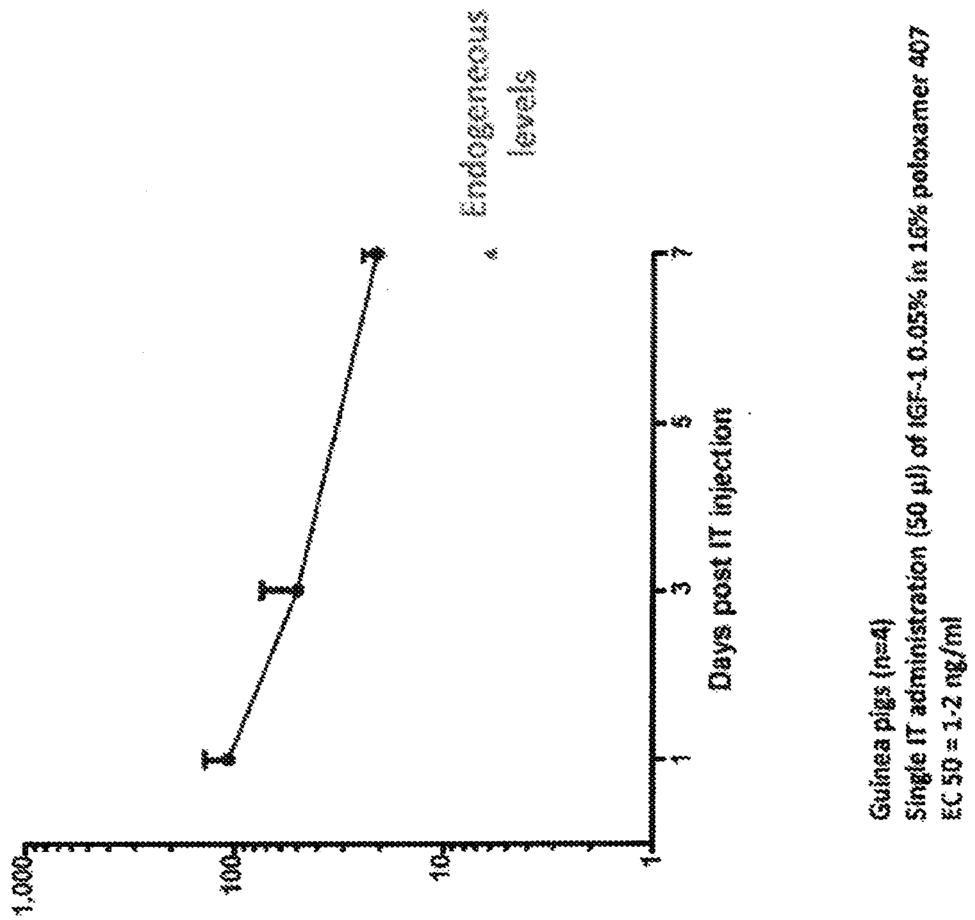
IGF-1 in perilymph [ng/ml]
1,000
100
10
1
1
3
5
7
Days post IT injection
Endogenous levels
Guinea pigs (n=4)
Single IT administration (50 µl) of IGF-1 0.05% in 16% poloxamer 407
EC 50 = 1.2 ng/ml

FIG. 8

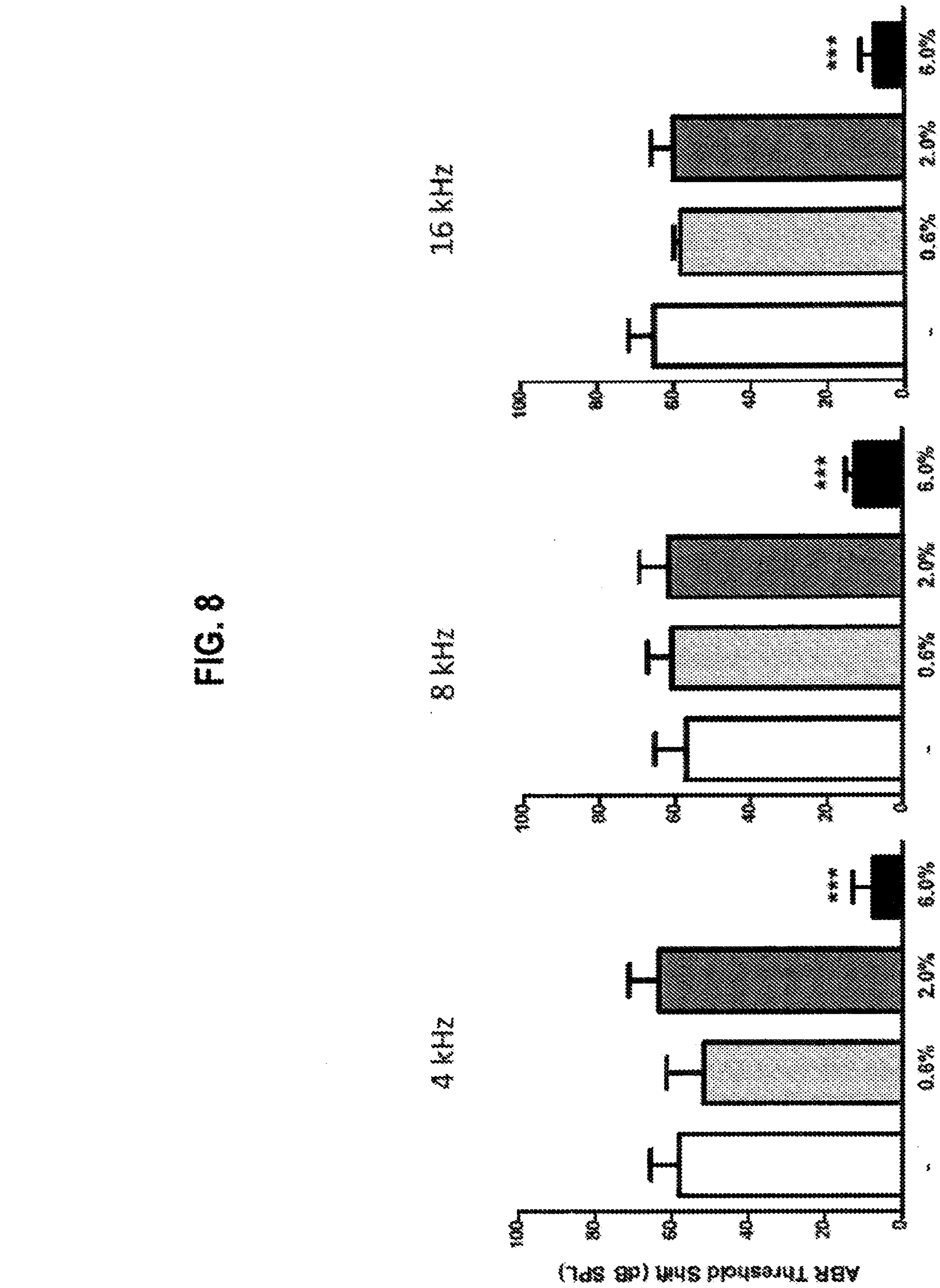
4 kHz
8 kHz
16 kHz
ABR Threshold Shift (dB SPL)
100
80
60
40
20
0
-
0.6%
2.0%
6.0%
***

FIG. 9

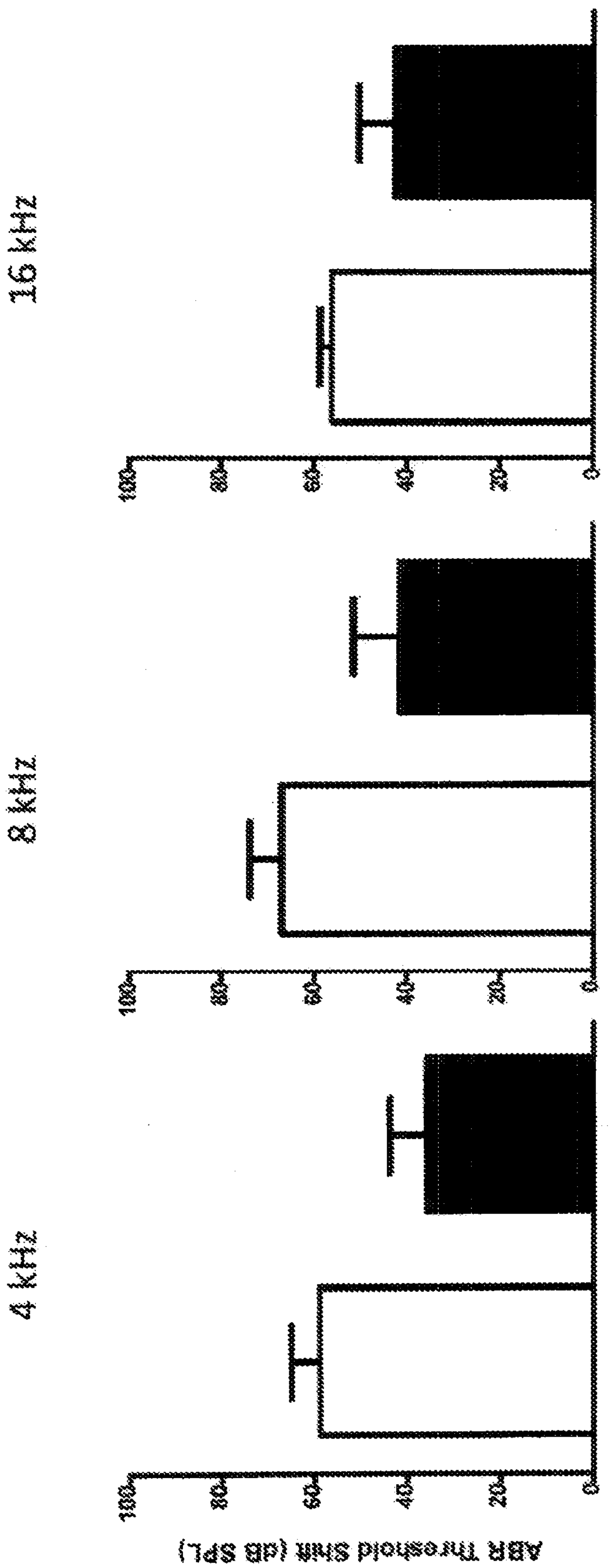

FIG. 10

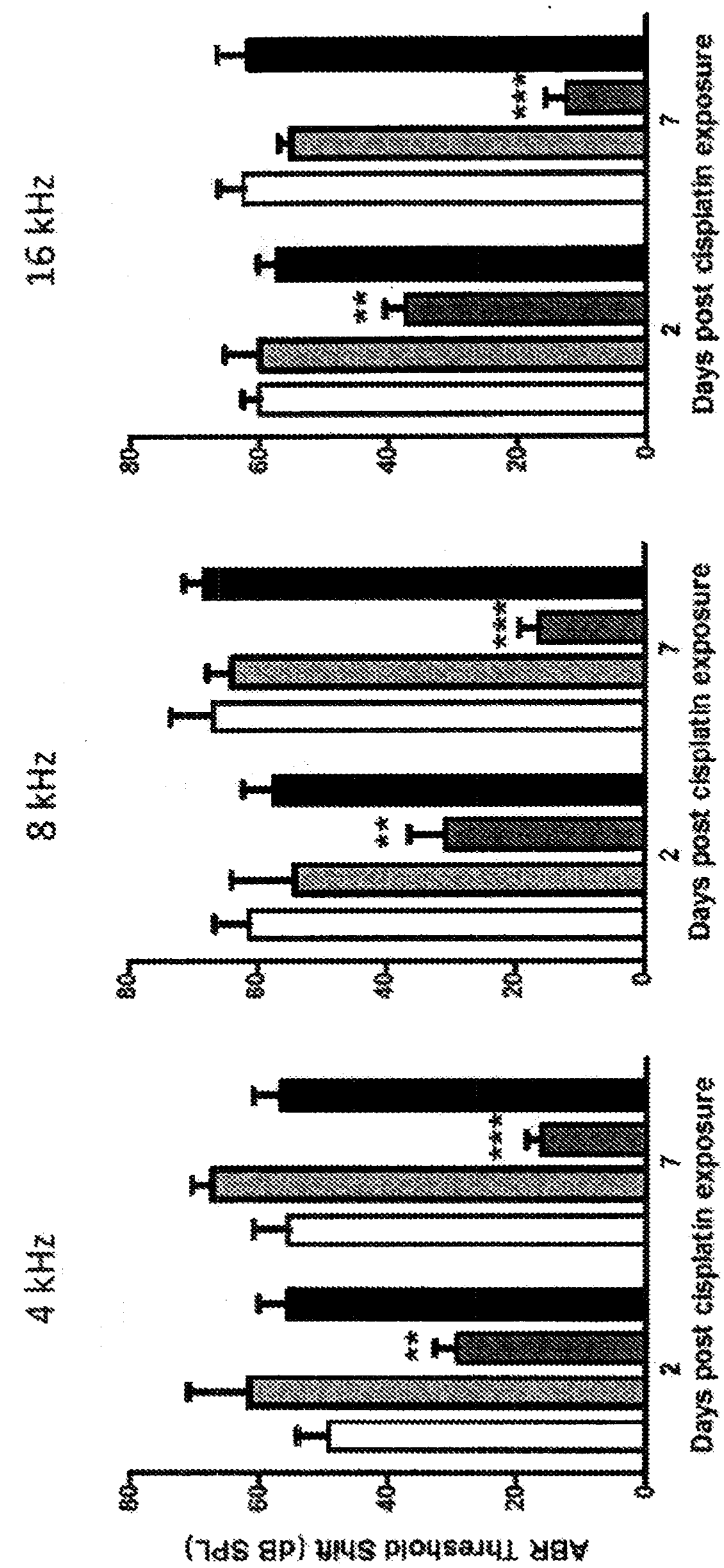
4 kHz
8 kHz
16 kHz
ABR Threshold Shift (dB SPL)
80
60
40
20
0
**
***
2
7
Days post cisplatin exposure

FIG. 11

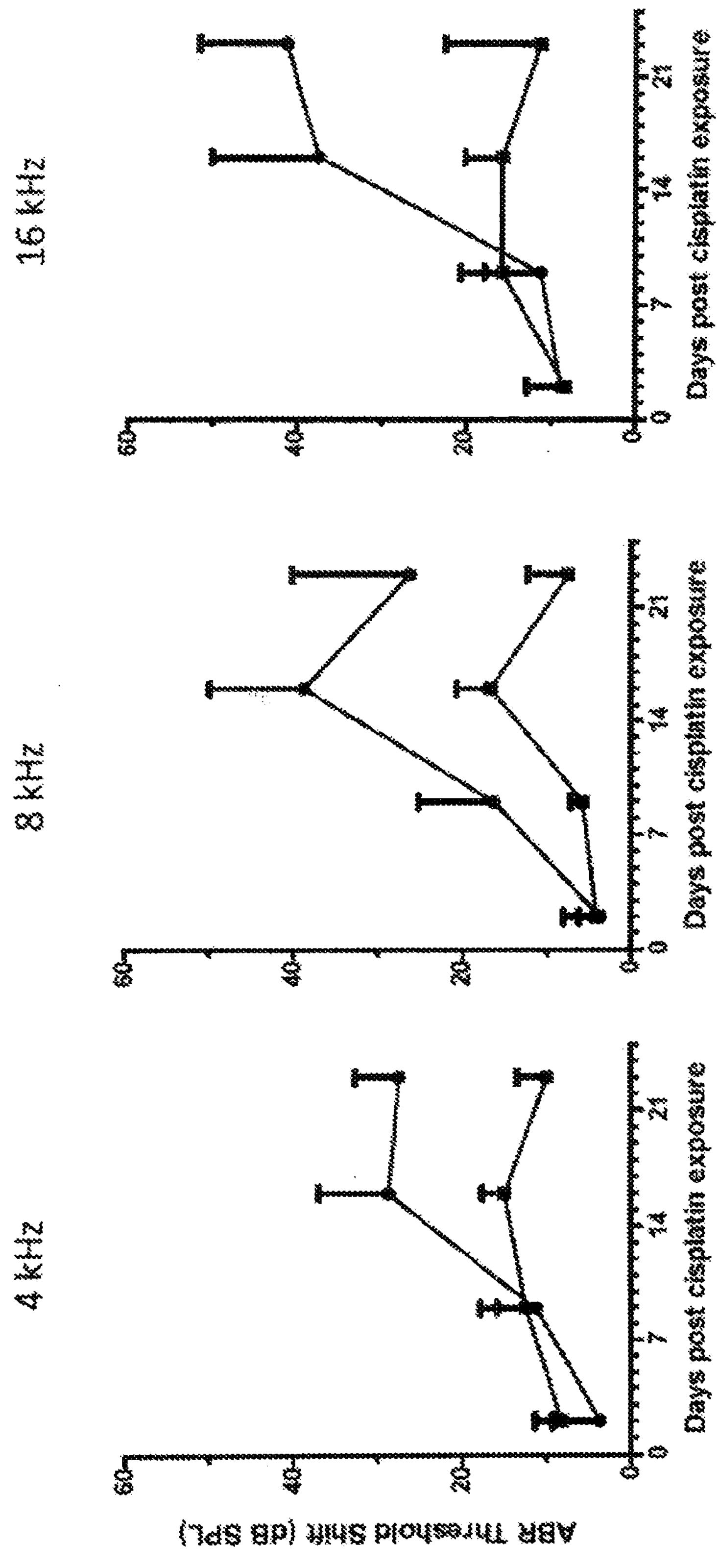
4 kHz
8 kHz
16 kHz
ABR Threshold Shift (dB SPL)
Days post cisplatin exposure
0
7
14
21
0
20
40
60

Europäisches Patentamt
European Patent Office
Office européen des brevets

# EUROPEAN SEARCH REPORT

Application Number
EP 18 17 4583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2010/009952 A1 (LICHTER JAY [US] ET AL) 14 January 2010 (2010-01-14)<br>* claims 1, 2, 7, 14, 15, 19-22 *<br>* paragraphs [0112], [0133] - [0135], [0217] * | 1-3,7,14<br><br>1-15 | INV.<br>A61K31/573<br>A61K31/65<br>A61K31/282<br>A61K31/4439<br>A61K31/506<br>A61K31/416<br>A61K31/24<br>A61K9/10<br>A61K47/10<br>A61P27/16 |
| X<br><br>Y | US 2010/016218 A1 (LICHTER JAY [US] ET AL) 21 January 2010 (2010-01-21)<br>* claims 1, 4, 7, 8, 10, 11, 13, 14, 18 *<br>* paragraphs [0161] - [0164], [0181] - [0182], [0373] * | 1-4,6-14<br><br>1-15 | |
| Y | US 2010/015263 A1 (LICHTER JAY [US] ET AL) 21 January 2010 (2010-01-21)<br>* claims 9-12 *<br>* paragraphs [0298], [0231], [0434] * | 1-15 | |
| Y | HILL GERHARD W ET AL: "Cisplatin-induced ototoxicity: effect of intratympanic dexamethasone injections", OTOLOGY & NEUROTOLOGY : OFFICIAL PUBLICATION OF THE AMERICAN OTOLOGICAL SOC, AMERICAN NEUROTOLOGY SOCIETY [AND] EUROPEAN ACADEMY OF OTOLOGY AND NEUROTOLOGY, vol. 29, no. 7, October 2008 (2008-10), pages 1005-1011, XP009182840, ISSN: 1537-4505, DOI: 10.1097/MAO.0B013E31818599D5<br>* abstract *<br>* figures 2, 3 * | 1-15 | TECHNICAL FIELDS SEARCHED (IPC)<br>A61K<br>A61P |

-/--

The present search report has been drawn up for all claims

1

| Place of search | Date of completion of the search | Examiner |
|---|---|---|
| Munich | 15 May 2019 | Peris Antoli, Berta |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 18 17 4583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PAKSOY MUSTAFA ET AL: "The protective effects of intratympanic dexamethasone and vitamin E on cisplatin-induced ototoxicity are demonstrated in rats", MEDICAL ONCOLOGY, SPRINGER-VERLAG, NEW YORK, vol. 28, no. 2, 19 March 2010 (2010-03-19), pages 615-621, XP019909553, ISSN: 1559-131X, DOI: 10.1007/S12032-010-9477-4 * abstract * * tables 2, 3 * * figure 3 * | 1-15 | |
| Y | HIMENO CHIEMI ET AL: "Intra-cochlear administration of dexamethasone attenuates aminoglycoside ototoxicity in the guinea pig", HEARING RESEARCH, vol. 167, no. 1-2, May 2002 (2002-05), pages 61-70, XP055172496, ISSN: 0378-5955, DOI: 10.1016/S0378-5955(02)00345-3 * abstract * | 1-15 | TECHNICAL FIELDS SEARCHED (IPC) |
| Y | US 2003/108539 A1 (BONNY CHRISTOPHE [CH]) 12 June 2003 (2003-06-12) * claims 1, 2, 7, 8, 10-12 * * paragraphs [0011], [0012] * | 1-15 | |
| | -/-- | | |

The present search report has been drawn up for all claims

| Place of search | Date of completion of the search | Examiner |
|---|---|---|
| Munich | 15 May 2019 | Peris Antoli, Berta |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 18 17 4583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PIRVOLA U ET AL: "Rescue of hearing, auditory hair cells, and neurons by CEP-1347/KT7515, an inhibitor of c-Jun N-terminal kinase activation", JOURNAL OF NEUROSCIENCE, vol. 20, no. 1, January 2000 (2000-01), pages 43-50, XP055172070, ISSN: 0270-6474 * abstract * ----- | 1-15 | |
| Y | RYBAK L P ET AL: "Ototoxicity: therapeutic opportunities", DRUG DISCOVERY TODAY, ELSEVIER, AMSTERDAM, NL, vol. 10, no. 19, October 2005 (2005-10), pages 1313-1321, XP027684992, ISSN: 1359-6446 [retrieved on 2005-10-01] * table 1 * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 15 May 2019 | Peris Antoli, Berta |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 18 17 4583

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☒ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

## ANNEX TO THE EUROPEAN SEARCH REPORT ON EUROPEAN PATENT APPLICATION NO. EP 18 17 4583

This annex lists the patent family members relating to the patent documents cited in the above-mentioned European search report. The members are as contained in the European Patent Office EDP file on
The European Patent Office is in no way liable for these particulars which are merely given for the purpose of information.

15-05-2019

| Patent document cited in search report | Publication date | Patent family member(s) | Publication date |
|---|---|---|---|
| US 2010009952 A1 | 14-01-2010 | AU 2009246870 A1 | 19-11-2009 |
| | | CA 2723458 A1 | 19-11-2009 |
| | | CN 102026623 A | 20-04-2011 |
| | | CN 103417472 A | 04-12-2013 |
| | | EP 2303227 A2 | 06-04-2011 |
| | | HK 1156533 A1 | 17-04-2014 |
| | | IL 208806 A | 29-02-2016 |
| | | JP 5551685 B2 | 16-07-2014 |
| | | JP 6014069 B2 | 25-10-2016 |
| | | JP 2011529854 A | 15-12-2011 |
| | | JP 2014101392 A | 05-06-2014 |
| | | JP 2016153423 A | 25-08-2016 |
| | | KR 20100135321 A | 24-12-2010 |
| | | KR 20130100017 A | 06-09-2013 |
| | | MY 161021 A | 31-03-2017 |
| | | RU 2010150863 A | 20-06-2012 |
| | | RU 2012139802 A | 27-03-2014 |
| | | US 2010009952 A1 | 14-01-2010 |
| | | US 2011008456 A1 | 13-01-2011 |
| | | US 2011319373 A1 | 29-12-2011 |
| | | US 2011319374 A1 | 29-12-2011 |
| | | US 2011319375 A1 | 29-12-2011 |
| | | US 2011319377 A1 | 29-12-2011 |
| | | US 2014364403 A1 | 11-12-2014 |
| | | US 2015313838 A1 | 05-11-2015 |
| | | US 2018110727 A1 | 26-04-2018 |
| | | WO 2009139924 A2 | 19-11-2009 |
| | | ZA 201007482 B | 25-01-2012 |
| US 2010016218 A1 | 21-01-2010 | AU 2009271129 A1 | 21-01-2010 |
| | | BR PI0915770 A2 | 03-11-2015 |
| | | CA 2730847 A1 | 21-01-2010 |
| | | CN 102099013 A | 15-06-2011 |
| | | EP 2296632 A2 | 23-03-2011 |
| | | JP 5491502 B2 | 14-05-2014 |
| | | JP 2011528036 A | 10-11-2011 |
| | | KR 20110025867 A | 11-03-2011 |
| | | RU 2011105257 A | 20-08-2012 |
| | | US 2010016218 A1 | 21-01-2010 |
| | | US 2016199446 A1 | 14-07-2016 |
| | | WO 2010008995 A2 | 21-01-2010 |
| | | ZA 201008861 B | 28-09-2011 |
| US 2010015263 A1 | 21-01-2010 | US 2010015263 A1 | 21-01-2010 |
| | | US 2010273864 A1 | 28-10-2010 |
| | | US 2014018395 A1 | 16-01-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 17 4583

This annex lists the patent family members relating to the patent documents cited in the above-mentioned European search report. The members are as contained in the European Patent Office EDP file on
The European Patent Office is in no way liable for these particulars which are merely given for the purpose of information.

15-05-2019

| Patent document cited in search report | Publication date | Patent family member(s) | Publication date |
|---|---|---|---|
| | | US 2015313839 A1 | 05-11-2015 |
| | | US 2016038491 A1 | 11-02-2016 |
| | | WO 2010062413 A1 | 03-06-2010 |
| US 2003108539 A1 | 12-06-2003 | AT 372125 T | 15-09-2007 |
| | | AT 417623 T | 15-01-2009 |
| | | AU 2003274820 A1 | 22-12-2003 |
| | | CA 2488695 A1 | 18-12-2003 |
| | | CY 1108847 T1 | 02-07-2014 |
| | | CY 1114715 T1 | 05-10-2016 |
| | | CY 1115331 T1 | 04-01-2017 |
| | | DK 1776958 T3 | 06-04-2009 |
| | | DK 1911458 T3 | 07-04-2014 |
| | | EP 1511507 A1 | 09-03-2005 |
| | | EP 1776958 A2 | 25-04-2007 |
| | | EP 1911458 A2 | 16-04-2008 |
| | | EP 1970070 A2 | 17-09-2008 |
| | | EP 2060265 A2 | 20-05-2009 |
| | | EP 2532357 A2 | 12-12-2012 |
| | | EP 2845603 A1 | 11-03-2015 |
| | | ES 2319454 T3 | 07-05-2009 |
| | | ES 2439950 T3 | 27-01-2014 |
| | | ES 2473274 T3 | 04-07-2014 |
| | | HK 1098961 A1 | 03-04-2009 |
| | | HK 1111635 A1 | 08-08-2014 |
| | | HK 1207971 A1 | 19-02-2016 |
| | | JP 4460445 B2 | 12-05-2010 |
| | | JP 2006501165 A | 12-01-2006 |
| | | JP 2010053136 A | 11-03-2010 |
| | | PT 1776958 E | 17-02-2009 |
| | | PT 1911458 E | 07-05-2014 |
| | | PT 2060265 E | 27-11-2013 |
| | | SI 1776958 T1 | 30-06-2009 |
| | | US 2003108539 A1 | 12-06-2003 |
| | | WO 03103698 A1 | 18-12-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61444413 A
- US 61514272 A
- WO 1998017285 A
- US 6004573 A
- US 6117949 A
- US 6201072 B
- US 6287588 B
- US 906041 A
- US 09559799 B
- US 10919603 B
- US 20080269449 A
- US 7148320 B
- US 5800711 A
- US 6977045 B
- US 6761824 B
- US 5567859 A
- WO 9216484 A
- US 5523492 A
- US 5696298 A
- US 6139870 A


### Non-patent literature cited in the description


- **RYBAK et al.** *Drug Disc. Today,* 2005, vol. 10, 1313-21
- **JEONG et al.** *Nature,* 1997, vol. 388, 860-2
- **JEONG et al.** *J. Control. Release,* 2000, vol. 63, 155-63
- **JEONG et al.** *Adv. Drug Delivery Rev.,* 2002, vol. 54, 37-51
- **VIEGAS.** *Int. J. Pharm.,* 1998, vol. 160, 157-162
- *Guidance for Industry: Sterile Drug Products Produced by Aseptic Processing, http://www.fda.gov/cder/guidance/5882fnl.htm*
- Remington: The Science and Practice of Pharmacy. Lippincott, Williams & Wilkins
- Microbiological Evaluation of Filters for Sterilizing Liquids. Health Industry Manufacturers Association, 1981, vol. 4
- **RICHARD et al.** *International Journal of Pharmaceutics,* 2006, vol. 312 (1-2), 144-50
- United States Pharmacopoeia (USP) <71> Sterility Tests. 1995
- United States Pharmacopeia
- USP23/NF 18, Biological Tests. The United States Pharmacopeial Convention, 1995
- **TAKTAK et al.** *J. Pharm. Pharmacol.,* 1990, vol. 43, 578-82
- **LI XIN-YU.** *Acta Pharmaceutica Sinica,* 2008, vol. 43 (2), 208-203